(19) Europäisches Patentamt
European Patent Office
Office européen des brevets

(11) **EP 3 446 621 B1**

(12) **FASCICULE DE BREVET EUROPEEN**

(45) Date de publication et mention de la délivrance du brevet:
**30.11.2022 Bulletin 2022/48**

(21) Numéro de dépôt: **18163318.1**

(22) Date de dépôt: **08.03.2013**

(51) Classification Internationale des Brevets (IPC):
**A61B 3/11** *(2006.01)*    **A61B 3/14** *(2006.01)*
**A61B 3/00** *(2006.01)*

(52) Classification Coopérative des Brevets (CPC):
**A61B 3/11; G02C 13/005**

(54) **PROCEDE DE DETERMINATION D'UNE CARACTERISTIQUE GEOMETRICO-MORPHOLOGIQUE, DE POSTURE OU COMPORTEMENTALE D'UN PORTEUR D'UNE PAIRE DE LUNETTES**

BESTIMMUNGSVERFAHREN EINER GEOMETRISCH-MORPHOLOGISCHEN, HALTUNGS- ODER VERHALTENS-CHARAKTERISTIK EINES BRILLENTRÄGERS

METHOD FOR DETERMINING A GEOMETRIC-MORPHOLOGICAL, POSTURAL OR BEHAVIOURAL CHARACTERISTIC OF A WEARER OF A PAIR OF SPECTACLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.03.2012 FR 1200705**

(43) Date de publication de la demande:
**27.02.2019 Bulletin 2019/09**

(62) Numéro(s) de document de la (des) demande(s) initiale(s) en application de l'article 76 CBE:
**13719888.3 / 2 822 449**

(73) Titulaire: **Essilor International**
**94220 Charenton-le-Pont (FR)**

(72) Inventeurs:
• **HADDADI, Ahmed**
**94227 Charenton-le-Pont, Cedex (FR)**
• **DELZERS, Jean**
**94227 Charenton-le-Pont, Cedex (FR)**

(74) Mandataire: **Jacobacci Coralis Harle**
**32, rue de l'Arcade**
**75008 Paris (FR)**

(56) Documents cités:
**EP-A1- 0 680 722        WO-A2-2009/024681**
**JP-B2- 3 176 147         US-A- 5 402 199**
**US-A1- 2006 044 509     US-A1- 2009 021 693**
**US-A1- 2010 128 220     US-A1- 2011 228 975**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

DOMAINE TECHNIQUE AUQUEL SE RAPPORTE L'INVENTION

La présente invention concerne de manière générale la prise de mesures sur un sujet.

Elle trouve une application particulière, mais non exclusive, à la prise de mesures sur un porteur de lunettes en vue de la conception optique personnalisée de lentilles ophtalmiques correctrices adaptées à ce porteur.

Elle concerne plus particulièrement un procédé et un dispositif de détermination d'au moins une caractéristique d'optimisation d'implantation d'une paire de lunettes sur le visage d'un porteur, ladite caractéristique d'optimisation étant une caractéristique géométrico-morphologique, de posture ou comportementale d'un porteur d'une paire de lunettes.

ARRIERE-PLAN TECHNOLOGIQUE

Lors de la conception d'une lentille ophtalmique correctrice, on cherche à prendre en compte un nombre important de paramètres individuels, dits de conception optique personnalisée, attachés au porteur et à la monture de lunettes sélectionnée, afin d'usiner la lentille de telle sorte qu'elle s'adapte au mieux au porteur.

Pour déterminer ces paramètres, l'opticien place la monture de lunettes sélectionnée sur le nez du porteur et réalise différentes opérations de mesure sur le porteur ainsi équipé. Ainsi peut-il notamment déterminer les demi-écarts pupillaires du porteur, c'est-à-dire les distances entre l'arête du nez du porteur et chacune des pupilles de ce dernier.

On connaît par exemple du document WO 2008/009423 un procédé pour détecter les positions de deux lentilles dans une monture de lunettes, qui utilise deux sources de lumière visible et deux capteurs d'images. Dans ce document, les lentilles sont repérées à l'aide d'autocollants collés sur leurs faces avant.

Les capteurs d'images sont situés face au porteur. Les deux sources de lumière sont quant à elles situées au-dessus du porteur de lunettes afin d'éclairer le porteur sans pour autant générer de reflets sur les lentilles qui perturberaient les mesures.

L'intensité lumineuse émise par les deux sources de lumière est alors choisie pour éclairer le visage du porteur de manière que l'image acquise soit correctement contrastée.

Ce procédé présente toutefois divers inconvénients.

Il est ainsi nécessaire de coller avec soin les autocollants sur les lentilles, ce qui nécessite un personnel formé à cet effet.

La localisation automatique des autocollants est difficile à mettre en œuvre et présente un taux d'échec significatif, si bien que l'opticien est souvent forcé de localiser manuellement ces autocollants sur l'image acquise.

L'image acquise fait apparaître les autocollants, ce qui est peu esthétique et donc peu flatteur pour le porteur de lunettes qui se voit dans un miroir et sur l'image acquise.

Les images acquises sont par ailleurs perturbées par l'ambiance lumineuse qui génère des reflets parasites sur les lentilles, susceptibles de cacher les autocollants.

Afin d'obtenir une image bien contrastée, l'intensité lumineuse choisie est généralement éblouissante pour le porteur. Il serait bien entendu possible d'utiliser un flash pour remédier à cet inconvénient, mais celui-ci provoquerait nécessairement un mouvement de recul du porteur susceptible de fausser les mesures.

Enfin, le procédé est inefficient pour le traitement des paires de lunettes solaires, sauf à démonter les lentilles teintées, ce qui est au mieux fastidieux, au pire impossible dans le cas des montures sans cercle.

Le document WO2009024681 divulgue un procédé de mesure de paramètres géométrico-physionomiques pour l'implantation d'une monture de lunettes sur le visage d'un porteur. Selon ce procédé, il est prévu de capturer une image numérique du visage du porteur équipé de sa monture à l'aide d'un appareil portable, puis de traiter cette image.

Le but exposé dans ce document est de permettre à l'opticien de capturer une image du porteur qui puisse être traitée facilement, en plaçant l'axe optique de l'appareil de capture d'images horizontalement, à hauteur des yeux du porteur de lunettes.

Pour cela, l'une des méthodes décrites consiste à projeter sur le visage du porteur une ligne horizontale au moyen d'une source de lumière montée sur l'appareil de capture d'images. Alors, l'opticien saura que l'appareil est en position lorsque ladite ligne passera par les pupilles des deux yeux du porteur. La source de lumière peut être constituée par un pointeur infrarouge.

Le document US5402199 décrit quant à lui un appareil de détection de l'axe du regard du porteur, qui comporte cinq diodes infrarouges pour éclairer l'œil du porteur de lunettes, un capteur d'images infrarouges, et un miroir réfléchissant la lumière infrarouge vers le capteur d'images infrarouges.

Dans ce document, il est prévu d'éclairer l'œil du porteur avec une partie des diodes infrarouges, d'acquérir une image du porteur, de distinguer parmi les reflets apparaissant sur l'image acquise ceux correspondant aux reflets cornéens, puis de déterminer l'axe du regard de cet œil en fonction de la position de la pupille et des reflets cornéens.

OBJET DE L'INVENTION

Afin de remédier aux inconvénients précités de l'état de la technique, la présente invention propose un procédé défini dans la revendication 1.

Grâce à l'invention, le porteur n'est pas ébloui par la source de lumière infrarouge lors de la prise des mesures.

Le capteur d'images infrarouges est en outre adapté à repérer facilement les positions :

- des reflets sur les lentilles de présentation, puisque les reflets parasites dans le domaine infrarouge sont réduits et qu'il est possible d'utiliser une intensité lumineuse forte, sans gêner le porteur, afin de distinguer les reflets de la source de lumière des reflets parasites, et
- des reflets sur les pupilles du porteur puisque, quand bien même les lentilles de la paire de lunettes seraient teintées, les pupilles du porteur seraient visibles en lumière infrarouge.

L'utilisation de traitements anti-reflets sur les lentilles n'est par ailleurs pas problématique dans la mesure où ces traitements ne sont pas conçus pour réduire les reflets dans le domaine de lumière infrarouge.

Par définition, on définira ici une caractéristique géométrico-morphologique comme une caractéristique relative à la forme du visage du porteur de lunettes.

Il pourra par exemple s'agir de l'écart entre les pupilles du porteur, de la distance entre chaque pupille du porteur et le bas du cercle correspondant de la monture de lunettes (on parle de « hauteur pupillaire »), de la distance entre chaque œil du porteur et la lentille correspondante de la monture de lunettes.

Par définition, on définira une caractéristique de posture comme une caractéristique relative à la position du porteur de lunettes dans le référentiel de mesure.

Il pourra par exemple s'agir de l'angle de lacet entre le plan sagittal de la tête du porteur et l'axe optique de la caméra, de l'angle de tangage entre le plan de Francfort de la tête du porteur et l'axe optique de la caméra, de l'angle de roulis de la tête du porteur autour de l'axe optique de la caméra.

Par définition, on définira une caractéristique comportementale comme une caractéristique relative aux réactions du porteur à un stimulus.

Il pourra par exemple s'agir de la propension du porteur à plutôt détourner les yeux ou la tête pour observer un objet venant d'entrer dans son champ de vision.

D'autres caractéristiques avantageuses et non limitatives du procédé de détermination conforme à l'invention sont les suivantes :

- à l'étape c), on localise également sur l'image acquise à l'étape b) les reflets de la source de lumière infrarouge réfléchis par les deux cornées du porteur ;
- à l'étape b), ladite image est acquise dans le domaine du proche infrarouge, avec une longueur d'onde comprise entre 780 et 1400 nm ;
- ladite caractéristique de posture comporte un angle de lacet, qui sépare le plan sagittal de la tête du porteur et l'axe optique du capteur d'images infrarouges ;
- ladite caractéristique géométrico-morphologique comporte les demi-écarts pupillaires du porteur ;
- ladite caractéristique géométrico-morphologique comporte les hauteurs pupillaires du porteur ;
- à l'étape b), le capteur d'images infrarouges acquiert au moins deux images de la tête du porteur, à l'étape c), on localise lesdits reflets sur chaque image acquise à l'étape b), et, à l'étape d), ladite caractéristique comportementale comporte un coefficient quantifiant la propension du porteur à détourner le regard en bougeant plutôt la tête ou les yeux ;
- ladite caractéristique géométrico-morphologique comporte les positions des centres de rotation des yeux du porteur.

L'invention concerne également un dispositif défini dans la revendication 9.

D'autres caractéristiques avantageuses et non limitatives du dispositif de détermination conforme à l'invention sont les suivantes :

- l'unité de calcul est formée par une unité centrale d'un ordinateur et dans lequel la source de lumière infrarouge et le capteur d'images infrarouges sont dissociés de l'unité de calcul et sont équipés de moyens de communication adaptés à communiquer avec l'unité centrale de l'ordinateur ;
- il est prévu un bâti auquel sont solidarisés la source de lumière infrarouge, le capteur d'images infrarouges et l'unité de calcul ;
- il est prévu une coulisse qui est montée mobile sur le bâti et qui supporte la source de lumière infrarouge et/ou le capteur d'images infrarouges ;
- il est prévu une unique source de lumière montée mobile par rapport audit capteur d'image ou au moins deux sources de lumière distantes l'une de l'autre pour générer deux reflets distincts sur ladite image ;

- il est prévu des moyens de motorisation de la mobilité de la coulisse, et dans lequel ladite unité de calcul est adaptée à piloter automatiquement lesdits moyens de motorisation dans une position telle que le visage dudit individu soit visible par le capteur d'images infrarouges ;
- chaque source de lumière infrarouge est composée d'au moins une diode électroluminescente ;
- le capteur d'images infrarouges est également adapté à acquérir des images dans le domaine visible ;
- il est prévu un capteur d'images visibles, distinct du capteur d'images infrarouges, qui est adapté à acquérir une image du porteur dans le domaine visible ;
- il est prévu des moyens d'affichage et/ou d'impression des images visibles acquises ; et
- il est prévu des moyens d'acquisition d'au moins une caractéristique géométrique de la monture de lunettes.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est une vue schématique en perspective de la tête d'un porteur de lunettes ;
- la figure 2 est une vue schématique de dessus de la tête du porteur de lunettes de la figure 1 ;
- la figure 3 est un schéma sur lequel apparaissent les yeux du porteur de lunettes de la figure 1 et un capteur d'images ;
- la figure 4 est une vue schématique en perspective d'un dispositif adapté à mettre en œuvre le procédé selon l'invention ;
- les figures 5 à 7 sont des représentations de trois images acquises par le capteur d'images de la figure 3 ; et
- la figure 8 est une vue schématique de dessus de la tête du porteur de lunettes de la figure 1.

Dans la description qui va suivre, certaines références auront un numéro suivi de la lettre G ou D. Ces références désigneront alors respectivement des éléments situés à gauche ou à droite par rapport au porteur de lunettes. Les yeux gauche et droit du porteur seront ainsi respectivement référencés 20G et 20D.

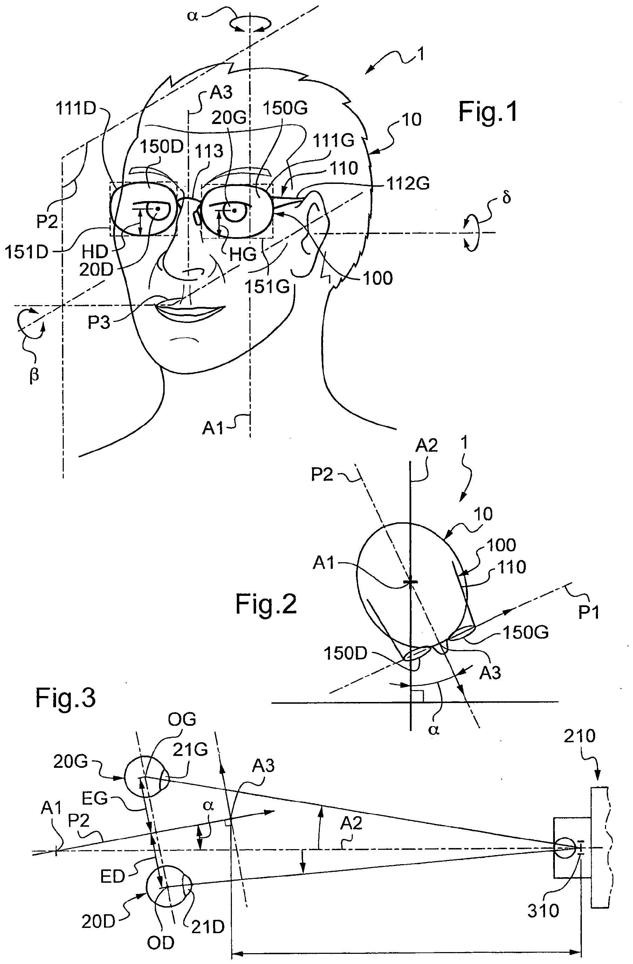

Sur la figure 1, on a représenté la tête 10 d'un individu 1 portant une paire de lunettes 100. Cet individu sera désigné dans la suite de cet exposé comme le « porteur ».

Dans cet exposé, on considérera également un autre individu, qui sera désigné comme le « vendeur », et qui aidera le porteur 10 à choisir une paire de lunettes 100 et à mesurer les données nécessaires à la fabrication des lentilles ophtalmiques (non représentées) en vue de leur commande et de leur montage dans la monture de lunettes 100 sélectionnée par le porteur.

Grâce à l'automatisation des mesures, ce vendeur ne sera pas nécessairement un opticien.

Préalablement aux mesures, le porteur choisira une paire de lunettes 100 parmi les paires de lunettes mises à disposition par le vendeur.

Lors des mesures, le porteur portera cette paire de lunettes 100.

Il sera positionné dans une configuration assise ou debout, avec sa tête 10 sensiblement droite et dirigée vers un capteur d'images 210 (voir figure 3).

Le vendeur demandera par ailleurs au porteur 1 de regarder une cible 310 située à proximité du capteur d'images 210 (voir figure 3). Les centres des deux pupilles 21G, 21D et les centres de rotation OD, OG des deux yeux 20G, 20D du porteur 1 seront donc respectivement alignés avec la cible 310.

Sur la figure 1, on observe que la paire de lunettes 100 choisie par le porteur 1 est du type cerclée (en variante, elle pourrait bien entendu être d'une autre sorte, telle que semi-cerclée ou percée).

Cette paire de lunettes 100 comporte une monture de lunettes 110, avec deux cercles 111G, 111D (ou entourages) dans lesquels sont emboîtées deux lentilles de présentation 150G, 150D (destinées à être remplacées par les lentilles ophtalmiques adaptées à l'acuité visuelle du porteur).

Les deux cercles 111G, 111D sont reliés l'un à l'autre par un pont ou pontet 113 équipé de deux plaquettes nasales reposant sur le nez du porteur. Ils sont également chacun équipés d'une branche 112G, 112D reposant sur les oreilles correspondantes du porteur 1. Ces branches 112G, 112D s'étendent chacune de manière rectiligne sur leur majeure partie, selon un axe longitudinal, et sont recourbées à leurs extrémités.

Chacun des deux cercles 111G, 111D de la monture de lunettes 110 présente, en creux dans son flanc intérieur, une rainure communément appelée drageoir dans laquelle est emboîté un biseau qui s'étend en saillie de la tranche de la lentille de présentation 150G, 150D correspondante.

Comme le montre la figure 2, on définit ici par rapport à la monture de lunettes 110 un plan moyen P1 qui passe au plus près de l'ensemble des points de l'arête de fond des drageoirs des deux cercles 111G, 111D.

Ce plan moyen P1 est incliné par rapport au plan passant par les axes longitudinaux des branches 112G, 112D d'un angle appelé « angle pantoscopique ». L'angle pantoscopique moyen d'une monture de lunettes est de l'ordre de 10 degrés.

Comme le montre la figure 1, sur chaque image du porteur 1 acquise par le capteur d'images 210, on peut repérer les cercles 111G, 111D de la monture de lunettes 110 au moyen de deux cadres appelés « cadres boxing » 151G, 151D.

Ces cadres boxing 151G, 151D sont définis comme les rectangles circonscrits aux cercles 111G, 111D de la monture de lunettes 110 (ou aux lentilles de présentation 150G, 150D), dont deux des côtés sont verticaux et dont les deux autres sont horizontaux.

On définit alors sur chaque image un axe remarquable A3 de la monture de lunettes 100, comme étant l'axe qui est parallèle aux côtés verticaux des cadres boxing 151G, 151D, et qui est situé à égale distance de ces derniers.

Comme le montre la figure 1, on définit par rapport au porteur 1 en position de mesure un plan de Francfort P3 comme étant le plan passant par les points orbitaires inférieurs et le porion du porteur (le porion étant le point du crâne le plus élevé du conduit auditif, qui correspond au tragion de l'oreille). En position de mesure, ce plan de Francfort P3 sera donc sensiblement horizontal.

On définit également un plan sagittal P2 comme étant le plan orthogonal au plan de Francfort P3 et à l'axe passant par les centres de rotation OG, OD des deux yeux 20G, 20D du porteur 1, passant par l'arête du nez du porteur. Lors des mesures, ce plan sagittal P2 sera donc sensiblement vertical. Sa position sera déduite non pas de la position du nez du porteur 1, mais plutôt en fonction de la position de la paire de lunettes 100 portée par le porteur 1.

On définit par rapport à la tête 10 du porteur 1 un axe longitudinal A1 comme étant orthogonal au plan de Francfort P3, compris dans le plan sagittal P2, et correspondant à l'axe de rotation de la tête 10 du porteur 1 lorsque ce dernier fait un mouvement de tête de droite à gauche (c'est-à-dire lorsque le porteur fait Non de la tête). En position de mesure, cet axe longitudinal A1 sera sensiblement vertical.

On définit également un axe frontal A4 comme étant l'axe d'intersection entre le plan de Francfort P3 et le plan sagittal P2.

On définit aussi un axe transversal A5 comme étant l'axe perpendiculaire aux axes longitudinal A1 et frontal A4.

En position de mesure, ces deux axes frontal et transversal A4, A5 seront sensiblement horizontaux.

Comme le montre la figure 3, les demi-écarts pupillaires EG, ED du porteur 1 sont définis comme les distances séparant le plan sagittal P2 des centres de rotation OG, OD des deux yeux 20G, 20D du porteur 1.

Comme le montre la figure 1, les hauteurs pupillaires HG, HD du porteur sont quant à elles définies comme les distances séparant, sur chaque image acquise, les centres de rotation OG, OD des yeux 20G, 20D du porteur 1 (qui sont en pratique confondus sur l'image avec les centres des pupilles 21G, 21D du porteur 1) et le bord inférieur du cadre boxing 151G, 151D correspondant.

Tel que représenté sur la figure 3, on définit par ailleurs par rapport au capteur d'images 210 un plan horizontal et un plan vertical, dont l'intersection est confondue avec l'axe optique A2 du capteur d'images 210.

Idéalement, on cherche alors à placer la tête 10 du porteur 1 de manière que son plan de Francfort P3 se confonde avec le plan horizontal du capteur d'images 210 et que son plan sagittal P2 se confonde avec le plan vertical du capteur d'images 210.

En pratique, on constate généralement un léger décalage entre ces plans, susceptible de fausser les mesures.

Ce décalage peut être mesuré au moyen de trois angles de roulis $\beta$, de tangage $\delta$ et de lacet $\alpha$, qui correspondent aux trois mobilités de pivotement de la tête 10 du porteur 1.

L'angle de lacet $\alpha$ sera ainsi défini comme l'angle de pivotement de la tête 10 du porteur 1 autour de l'axe longitudinal A1, entre la position idéale de la tête 10 du porteur (face à l'axe optique A2) et la position réelle de la tête 10 du porteur. Cet angle de lacet $\alpha$ sera mesuré dans le plan horizontal du capteur d'images 210.

L'angle de roulis $\beta$ sera défini comme l'angle de pivotement de la tête 10 du porteur 1 autour de l'axe frontal A4, entre la position idéale de la tête 10 du porteur et la position réelle de la tête 10 du porteur. Cet angle de roulis $\beta$ sera facilement mesurable sur chaque image acquise, en fonction de l'inclinaison de l'axe remarquable A3.

L'angle de tangage $\delta$ sera défini comme l'angle de pivotement de la tête 10 du porteur 1 autour de l'axe transversal A5, entre la position idéale de la tête 10 du porteur (face à l'axe optique A2) et la position réelle de la tête 10 du porteur. Cet angle de tangage $\delta$ sera mesuré dans le plan vertical du capteur d'images 210.

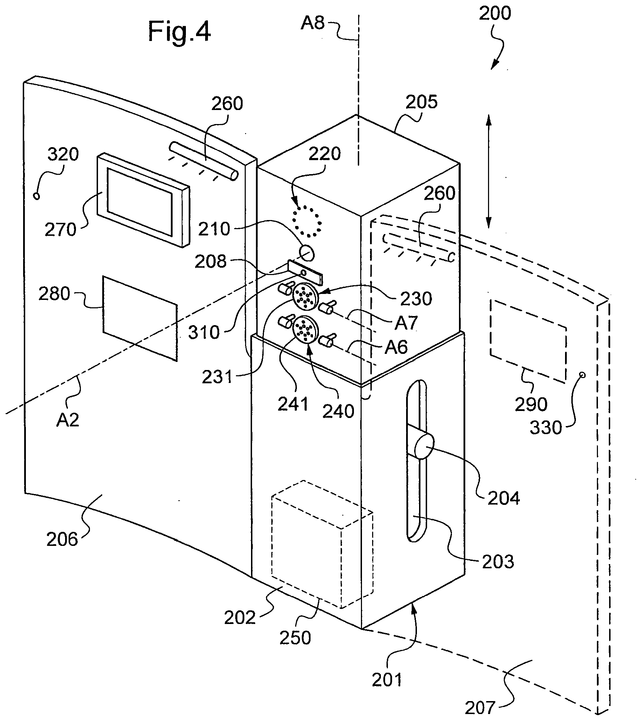

Sur la figure 4, on a représenté le dispositif 200 qui permet de mettre en oeuvre le procédé selon l'invention et qui, plus généralement, permet de mesurer et d'acquérir les données nécessaires à la commande des lentilles ophtalmiques à monter dans la monture de lunettes 110 sélectionnée par le porteur 1, en lieu et place des lentilles de présentation 150G, 150D.

Ce dispositif se présente sous la forme d'un kiosque à lunettes 200, faisant simultanément office de présentoir de montures de lunettes et de centre de mesure.

Il comporte à cet effet au moins un châssis 201, une source de lumière 220, 230, 240, un capteur d'images 210 et une unité de calcul 250.

De manière préférentielle, la source de lumière 220, 230, 240 émet dans l'infrarouge et le capteur d'images 210 capte des images infrarouges.

Le domaine de l'infrarouge qui sera ici utilisé est le proche infrarouge, dont les longueurs d'onde sont comprises entre 780 et 1400 nm.

L'utilisation d'une lumière infrarouge présente en effet plusieurs avantages. Elle permet notamment de s'affranchir des reflets parasites, provenant de la lumière extérieure ou de la lumière intérieure qui se réfléchit sur les lentilles de présentation 150G, 150D de la paire de lunettes 100. Elle permet aussi d'éviter au porteur 1 d'être ébloui lors des mesures. Elle permet enfin de voir les yeux du porteur 1 sur les images acquises par le capteur d'images 210, même lorsque les lentilles de présentation 150D, 150G sont teintées.

Le châssis pourrait être réalisé d'une seule pièce, de telle manière que le capteur d'images et les sources de lumière soient immobiles par rapport au sol sur lequel le kiosque à lunettes repose. Dans cette éventualité, il serait nécessaire de prévoir un tabouret réglable en hauteur, de manière à pouvoir placer le porteur de telle sorte que sa tête entre dans le champ du capteur d'images.

Au contraire, dans le mode de réalisation du kiosque 200 représenté sur la figure 4, le châssis 201 comprend un pied 202 qui est posé sur le sol, et une coulisse 205 qui est montée mobile en translation sur le pied 202 suivant un axe vertical A8 et qui supporte les sources de lumière 220, 230, 240 et le capteur d'images 210.

La position du capteur d'images 210 est ainsi réglable en hauteur en fonction de la taille du porteur 1.

Le pied 202 présente plus précisément une forme de parallélépipède creux, allongé suivant l'axe vertical A8 et de section horizontale carrée.

Ce pied 202 présente une extrémité inférieure fermée par une paroi posée au sol, et une extrémité supérieure ouverte par laquelle s'engage la coulisse 205.

Cette coulisse 205 présente à cet effet une forme parallélépipédique creuse, avec une section horizontale de dimensions extérieures égales aux dimensions intérieures du pied 202, ce qui lui permet de coulisser dans le pied 202.

Cette coulisse 205 présente une extrémité inférieure ouverte vers l'intérieur du pied 202, et une extrémité supérieure fermée par une paroi plane.

La coulisse 205 présente, sur deux côtés opposés, deux ergots 204 en saillie engagés dans deux trous oblongs verticaux 203 pratiqués dans deux côtés opposés du pied 202, pour permettre le guidage de la coulisse 205 en translation suivant l'axe vertical A8, entre deux positions de butée haute et basse.

Il est par ailleurs prévu des moyens d'actionnement motorisés (non représentés) permettant de monter ou descendre la coulisse 205 dans le pied 202, à la hauteur souhaitée.

Il est également prévu des moyens de détermination (non représentés) de la hauteur de la coulisse 205 dans le pied 202 (par exemple une roue codeuse associée à une crémaillère).

Le châssis 200 présente par ailleurs deux ailes latérales 206, 207 qui bordent sa face avant tournée vers le porteur 1.

Ces deux ailes latérales 206, 207 sont formées par des parois verticales légèrement incurvées vers l'avant et sont montées sur charnière sur les deux côtés opposés du pied 202 où sont pratiqués les trous oblongs verticaux 203.

Les faces avant de ces ailes 206, 207 sont par ailleurs équipées de crochets (non représentés) sur lesquels reposent les montures de lunettes parmi lesquelles le porteur fait son choix.

Sur la figure 4, on observe que la coulisse 205 porte au moins deux (ici trois) sources de lumière 220, 230, 240 infrarouges adaptées à éclairer la tête 10 du porteur 1 en position de mesure.

Ces sources de lumière 220, 230, 240 sont préférentiellement réparties de part et d'autre du capteur d'image, dans le plan vertical du capteur d'images 210.

Ces trois sources de lumière 220, 230, 240 sont ici montées fixes en translation sur la coulisse 205 et sont formées d'une pluralité de diodes électroluminescentes (LED).

L'une des sources de lumière, appelée source principale 220, est située à faible distance du capteur d'images 210 (c'est-à-dire ici à moins de 10 centimètres du capteur d'images). Elle est composée d'une pluralité de LED réparties sur un cercle. Ces LED sont directement fixées à la coulisse 205. Telle que représentée sur la figure 4, cette source principale 220 est située au-dessus du capteur d'images 210.

Les deux autres sources de lumière, appelées sources secondaires 230, 240, sont situées l'une au-dessus de l'autre, en dessous du capteur d'images 210. Elles sont chacune composées d'un nombre de LED égal à celui de la source principale 220, réparties sur deux cercles concentriques. Elles émettent une lumière d'intensité inférieure à celle de la lumière émise par la source principale 220. Les intensités lumineuses émises par les sources secondaires 230, 240 sont, au même titre que celle émise par la source principale 220, réglables.

Ces deux sources secondaires 230, 240 sont fixées sur des socles 231, 241 montés mobiles en rotation sur la coulisse 205 autour de deux axes de rotation A6, A7 horizontaux distincts. Grâce à cette mobilité, il est possible de diriger manuellement ces sources secondaires 230, 240 vers la tête 10 du porteur 1.

Les trois sources de lumière 220, 230, 240 sont plus précisément conçues pour former des reflets distincts sur les lentilles de présentation 150G, 150D de la paire de lunettes 100 portée par le porteur 1.

Ainsi, lorsque toutes les sources de lumière 220, 230, 240 se reflètent sur les deux lentilles de présentation 150G, 150D, le capteur d'images 210 peut observer six reflets-verres.

L'utilisation de trois sources de lumière 220, 230, 240 permet au capteur d'image 201 de voir au moins une partie de ces reflets, quel que soit l'angle de tangage δ de la tête du porteur 1. On comprend en effet que lorsque le porteur 1 incline la tête vers le bas, les reflets vus par le capteur d'images 210 remontent sur les lentilles de présentation 150G, 150D jusqu'à en sortir.

Les trois sources de lumière 220, 230, 240 forment par ailleurs un unique reflet sur chaque œil du porteur 1, appelé reflet cornéen.

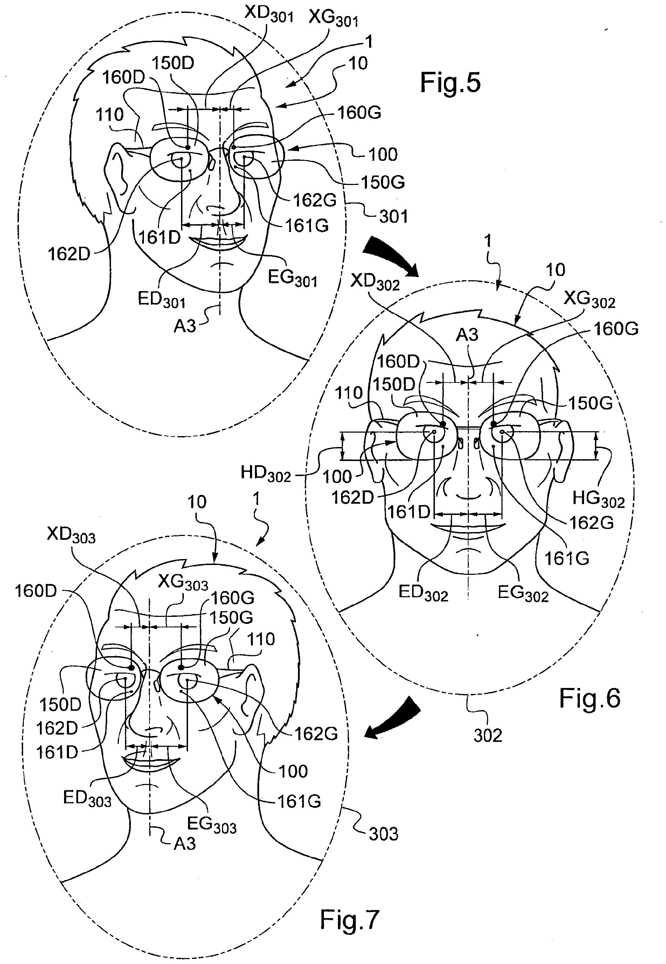

Sur la figure 6, on a représenté une situation dans laquelle seule deux des trois sources de lumière 220, 230, 240 se reflètent sur les deux lentilles de présentation 150G, 150D. On observe ainsi quatre reflets-verres 160G, 160D, 161G, 161D générés par la source principale 220 et l'une des sources secondaires 230 sur les deux lentilles de présentation 150G, 150D, et deux reflets cornéens 162G, 162D. La troisième source de lumière 240 est en revanche trop basse, eu égard à l'angle de tangage δ de la tête du porteur 1, pour générer des reflets sur les lentilles de présentation 150G, 150D.

Bien entendu, en variante, on pourrait prévoir que le kiosque ne comporte qu'une seule et unique source de lumière infrarouge.

Cette dernière pourrait être montée fixe à une position prédéterminée par rapport au capteur d'images. Il sera alors préférable d'utiliser une source de lumière de taille assez importante pour générer un reflet cornéen détectable. Cette source de lumière devra par ailleurs être placée de telle sorte que les deux reflets-verres qu'elle génère soient sensiblement situés à mi-hauteurs des lentilles de présentation 150G, 150D (c'est-à-dire sur l'axe passant par les centres des deux cadres boxing de la monture) lorsque la tête 10 du porteur 1 est idéalement placée (plan de Francfort P3 confondu avec le plan horizontal du capteur d'images 210 et plan sagittal P2 confondu avec le plan vertical du capteur d'images 210), compte tenu de l'angle pantoscopique moyen des montures.

Il conviendra alors de vérifier pendant les mesures que l'angle de tangage de la tête du porteur reste réduit, de sorte que le capteur d'image puisse voir les reflets de cette source de lumière sur les lentilles de présentation.

Selon une autre variante, on pourra prévoir que cette unique source de lumière soit montée mobile en translation verticale par rapport au capteur d'images, pour compenser l'angle de tangage de la tête du porteur.

Tel que le montre la figure 4, le kiosque 200 comporte ici un seul et unique capteur d'images 210, d'axe optique A2 horizontal.

Ce capteur d'image est ici formé par une caméra 210 adaptée à acquérir des images dans le proche infrarouge et dans le visible.

Cette caméra présente ici les références suivantes : Sony® FCB-EX490. Elle est équipée d'un filtre infrarouge basculant entre une position normale dans laquelle ce dernier filtre les infrarouges pour que la caméra 210 puisse acquérir une image dans le domaine du visible (appelée dans la suite « image-visible »), et une position escamotée dans laquelle il laisse les infrarouges passer jusqu'au capteur qui peut alors acquérir une image dans le domaine infrarouge (appelée dans la suite « image-infrarouge »).

Cette caméra est ainsi adaptée à acquérir des images-infrarouges du porteur sur lesquelles apparaissent nettement les reflets-verres et les reflets cornéens, ainsi que des images-visibles du porteur permettant à ce dernier de vérifier que la paire de lunettes 100 sélectionnée lui sied bien.

Bien entendu, en variante, il sera possible de prévoir non pas une seule caméra, mais deux caméras distinctes, l'une adaptée à acquérir des images-infrarouges et l'autre adaptée à acquérir des images-visibles.

Ici, la paroi avant de la coulisse 205 présente une fenêtre fermée par un miroir sans tain, à l'arrière duquel se trouve la caméra 210. La caméra 210 est ainsi invisible depuis l'extérieur du kiosque 200, mais elle demeure adaptée à acquérir des images des individus placés à l'avant du kiosque 200.

La caméra 210 est alors installée dans le kiosque 200 de telle manière que son objectif soit situé au contact ou à proximité de la face arrière de ce miroir sans tain.

L'objectif de la caméra 210 est par ailleurs entouré d'une paroi latérale opaque, permettant d'éviter que des reflets parasites n'apparaissent sur les images acquises.

En l'espèce, l'ensemble de la caméra est ici logé dans une boîte opaque qui est ouverte vers l'avant par une ouverture au travers de laquelle émerge l'objectif, cette boîte étant située au contact de la face arrière du miroir sans tain.

Le kiosque à lunettes 200 comporte par ailleurs au moins une cible 310 située sur le côté et à distance réduite de l'objectif de la caméra 210, de telle sorte qu'elle est visible par le porteur 1 en position de mesure.

Cette cible 310 comporte ici une fenêtre 208 pratiquée dans la paroi de la coulisse 205, entre l'objectif de la caméra 210 et les sources secondaires 230, 240, au travers de laquelle une LED est visible. En pratique, cette LED est ici située dans le pied 202 du châssis 200, et est réfléchie vers la fenêtre 208 au moyen d'un jeu de miroir.

Cette cible 310 permet d'attirer le regard du porteur 1 vers l'objectif de la caméra 210 lors des mesures.

Il est également prévu deux cibles supplémentaires 320, 330, situées sur les bords extérieurs de chacune des ailes 206, 207 du châssis 200, dans le plan horizontal de la caméra 210.

Comme cela sera bien exposé dans la suite de ce texte, ces cibles supplémentaires 320, 330 permettront d'attirer séquentiellement le regard du porteur 1 vers l'un des côtés du kiosque 200 puis l'autre, afin de déterminer si ce porteur 1 présente une propension à détourner le regard en bougeant plutôt la tête 10 ou les yeux 20G, 20D.

Il est par ailleurs prévu deux néons 260, respectivement positionnés sur les bords supérieurs des deux ailes 206, 207 du châssis 200, qui éclairent la tête 10 du porteur 1 de telle sorte que les images-visibles acquises par la caméra 210 soient bien exposées.

Il est aussi prévu dans l'une de ces deux ailes 206 des moyens d'affichage 270 et des moyens d'impression 280 des images-visibles acquises.

Les moyens d'affichage sont ici constitués par un écran tactile 270 fixé sur la face avant de l'aile 206, de manière à être visible par le porteur 1 en position de mesure.

Les moyens d'impression sont quant à eux conçus pour imprimer des fiches récapitulatives des données de commande des lentilles ophtalmiques, sur lesquelles apparaissent les photos des porteurs.

Il est par ailleurs prévu des moyens pour scanner les prescriptions du porteur 1.

Ici, les moyens d'impression et les moyens pour scanner sont confondus et sont constitués par une unique imprimante multifonction 280 couleur, située dans un logement prévu en creux dans la face avant de l'aile 206 du châssis 200.

Bien entendu, en variante, il serait également possible de prévoir deux appareils distincts, l'un pour imprimer les fiches récapitulatives et l'autre pour scanner les prescriptions.

Il est enfin prévu sur l'autre des deux ailes 207 un moyen d'acquisition d'au moins une dimension de la paire de lunettes 100. Les images acquises par la caméra 210 ne permettent en effet pas de déterminer les dimensions de la paire de lunettes 100 du porteur 1. Il est alors nécessaire de mettre à l'échelle les images acquises.

Les moyens d'acquisition 290 précités peuvent alors se présenter sous diverses formes.

De manière préférentielle, ils comportent un lecteur de code-barres 290 connecté à une base de données dont chaque enregistrement est associé à une référence de montures de lunettes et comprend un identifiant et des données relatives à cette référence de montures de lunettes.

En l'espèce, l'identifiant de chaque enregistrement est formé par le numéro du code-barres qui est assigné à la référence de montures de lunettes.

Les données mémorisées dans chaque enregistrement comportent quant à elles ici :

- la largeur totale des montures de lunettes de la référence considérée, mesurée entre les deux branches 112D, 112G,
- le rapport entre la hauteur et la largeur des cadres boxing 151G, 151D des cercles de ces montures de lunettes, et
- l'écart entre ces deux cadres boxing 151G, 151D.

Bien entendu, les données mémorisées dans chaque enregistrement de la base de donnée pourraient comporter un nombre réduit d'éléments (par exemple seulement la largeur totale de la monture de lunettes) ou un nombre accru d'éléments (par exemple la forme exacte des cercles, le matériau des montures de lunettes, l'angle pantoscopique et l'angle de galbe des montures de lunettes, ...).

En variante, ces moyens d'acquisition pourraient ne comporter qu'un simple clavier (physique ou affiché sur l'écran tactile) permettant au vendeur :

- de saisir la largeur de la monture de lunettes 110 sélectionnée, qu'il aura au préalable mesurée à l'aide d'un réglet, et
- de positionner sur l'image acquise deux curseurs au niveau des 2 points entre lesquels il aura mesuré la largeur de la monture.

Encore en variante, ces moyens d'acquisition pourraient ne comporter qu'un simple étalon de dimensions connues, à rapporter sur la monture de lunettes sélectionnée (par clipsage, collage ou tout autre moyen) pour obtenir sur chaque image acquise par la caméra une référence dont les dimensions sont connues, permettant ainsi de mettre cette image à l'échelle.

Comme le montre la figure 4, l'unité de calcul 250 du kiosque à lunettes 200 est quant à elle logée à l'intérieur du pied 202 du châssis 200. Elle est conçue pour piloter les différents composants électroniques du kiosque 200 et pour traiter les images acquises par la caméra 210.

Cette unité de calcul 250 comprend à cet effet un processeur (CPU), une mémoire vive (RAM), une mémoire morte (ROM), des convertisseurs analogiques-numériques (A/D), et différentes interfaces d'entrée, de sortie et de communication.

Grâce à ses interfaces d'entrée, l'unité de calcul 250 est adaptée à recevoir les images acquises par la caméra 210, la hauteur de la coulisse 205 dans le pied 202 mesurée par lesdits moyens de détermination, et le numéro de code-barres de la monture de lunettes sélectionnée, lu par le lecteur de code-barres 290.

Dans sa mémoire vive, l'unité de calcul 250 mémorise ainsi en continu ces différentes données.

Grâce à un logiciel enregistré dans sa mémoire morte, l'unité de calcul 250 est adaptée à mettre en oeuvre l'ensemble du procédé qui sera décrit dans la suite de cet exposé. Elle est ainsi par exemple adaptée à générer des signaux de pilotage des moyens d'actionnement de la bascule 205 pour positionner cette dernière à la hauteur souhaitée, et des signaux de communication comportant les données de commande des lentilles ophtalmiques.

Grâce à ses interfaces de sortie, l'unité de calcul 250 est adaptée à transmettre ces signaux de sortie aux différents composants électroniques du kiosque 200, notamment à la caméra 210, aux sources de lumière 220, 230, 240, à l'écran tactile 270, à l'imprimante multifonction 280, aux cibles 310, 320, 330 et aux moyens d'actionnement de la coulisse 205.

Grâce à ses interfaces de communication, l'unité de calcul 250 est adaptée à transmettre les signaux de communication à un centre de fabrication de lentilles ophtalmiques.Il est enfin prévu un interrupteur de mise sous tension du kiosque 200.

Préalablement à la venue du porteur 1, le vendeur met le kiosque 200 sous tension à l'aide de l'interrupteur prévu à cet effet.

Lors de cette mise sous tension, l'unité de pilotage commande l'alimentation électrique des néons 260, de manière à mettre en lumière les paires de lunettes qui sont à disposition sur les ailes 206, 207 du châssis 201 du kiosque 200.

Les trois sources de lumière 220, 230, 240 infrarouges restent quant à elles éteintes.

Puis, lorsqu'un individu se présente, après lecture d'un message sur l'écran l'y invitant, il choisit une paire de lunettes 100 parmi l'ensemble de celles qui sont en présentation sur les ailes 206, 207 du châssis 201 du kiosque 200. Dans l'exemple représenté sur les figures, la paire de lunettes sélectionnée est du type cerclé. Puis, comme l'y invite un message affiché sur l'écran, l'individu appelle le vendeur pour la suite du protocole.

Il est ensuite prévu une opération de détermination d'une dimension caractéristique de la monture de lunettes 110 sélectionnée.

Comme cela a été exposé supra, cette opération est notamment prévue pour permettre de mettre les images acquises à l'échelle.

Au cours de cette opération, le vendeur passe le code-barres de la monture de lunettes 110 sélectionnée devant le lecteur de code-barres 290. L'unité de calcul 250, grâce au numéro de code-barres, cherche alors dans la base de données l'enregistrement correspondant à cette monture de lunettes 110, puis elle récupère et mémorise dans sa mémoire morte les données suivantes : la largeur totale de la monture de lunettes 110, le rapport entre la hauteur et la largeur des cadres boxing 151G, 151D des cercles 111D, 111G de cette monture de lunettes 110, et l'écart entre ces deux cadres boxing 151G, 151D.

Bien entendu, comme cela a été exposé supra, cette opération de lecture de code-barres pourrait être remplacée par une opération plus simple consistant pour le vendeur à mesurer la largeur totale de la monture de lunettes à l'aide d'un réglet, à la saisir sur un clavier virtuel affiché sur l'écran tactile, et à positionner sur l'image acquise les deux curseurs au niveau des deux points entre lesquels il a mesuré la largeur de la monture (cette opération de positionnement des deux points pouvant en variante être réalisée automatiquement).

Il est alors prévu une opération d'acquisition des prescriptions du porteur 1, que ce dernier aura préalablement obtenues chez un optométriste.

Ces prescriptions sont généralement inscrites sur une feuille de prescription et comportent en particulier le type de lentilles (simples, bifocales, à variation progressive de puissances, teintées, ...) et le pouvoir de réfringence que devront présenter les lentilles pour corriger les déficiences de vision du porteur (c'est-à-dire leurs puissances optiques sphériques, cylindriques et prismatiques et leurs axes de cylindre). Elles peuvent bien sûr comporter d'autres informations telles que, dans le cas des lentilles bifocales ou à variation progressive de puissances, une addition.

Lors de cette opération, le vendeur recueille alors la feuille de prescription et la scanne à l'aide de l'imprimante multifonction 280, de manière que l'unité de calcul 250 puisse mémoriser l'image scannée de cette feuille de prescription dans sa mémoire morte.

Le vendeur peut également demander au porteur de choisir les traitements qu'il souhaite obtenir sur ses lentilles ophtalmiques (anti-reflets, hydrophobes, ...) et saisir ces informations dans des champs affichés à cet effet par l'unité de calcul 250 sur l'écran tactile 270.

Une fois ces informations acquises, l'unité de calcul 250 commande la mise sous tension de la première cible 310.

Elle commande également l'affichage sur l'écran tactile 270 :

- d'un message indiquant que les opérations de mesure peuvent commencer,
- des images acquises « en temps réel » par la caméra 210, et
- de deux flèches orientées à l'opposée l'une de l'autre, l'une vers le haut et l'autre vers le bas, pour piloter le déplacement de la coulisse 205 vers le haut ou vers le bas.

Le vendeur invite alors tout d'abord le porteur 1 à chausser ses lunettes et à les garder ainsi pendant l'ensemble des examens qui vont suivre.

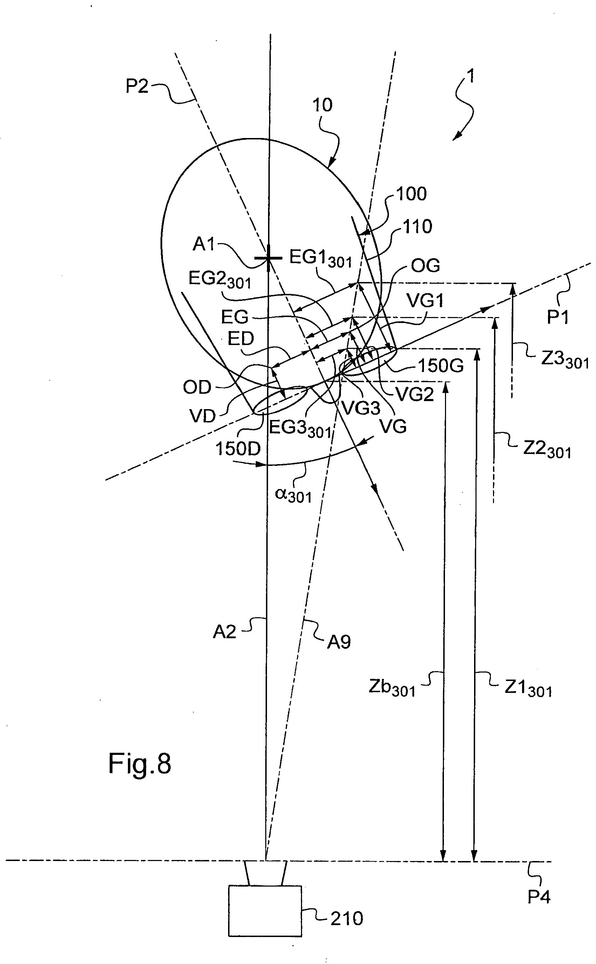

Comme cela sera exposé en détail dans la suite de cette description, ces examens permettront alors au kiosque 200 de déterminer les demi-écarts pupillaires EG, ED et les hauteurs pupillaires HG, HD du porteur 1, ainsi que des paramètres de personnalisation avancée tels que la distance VG, VD entre la monture de lunettes 110 et chaque œil du porteur 1 (figure 8), le comportement en mobilité de son regard...

Il demande pour cela au porteur 1 de se placer face au kiosque 200, en position de mesure, et de regarder la première cible 310 en gardant la tête droite, de telle manière que son plan de Francfort P3 soit sensiblement horizontal et que son plan sagittal P2 soit sensiblement vertical.

Ici, le porteur 1 est invité à se placer debout, face à la coulisse 205 du châssis 201 du kiosque 200.

Le vendeur règle alors la position de la coulisse 205 à une hauteur adaptée à la taille du porteur 1. Il utilise à cet effet les flèches affichées sur l'écran tactile 270 pour commander la montée ou la descente de la coulisse 205 jusqu'à une hauteur telle que l'ensemble du visage du porteur 1 apparaisse sur les images acquises par la caméra 210 et affichées sur l'écran tactile 270.

Ainsi, dans cette position, le visage du porteur 1 se trouve tout entier dans le champ de la caméra 210.

Cette opération de positionnement de la caméra 210 à la hauteur de la tête 10 du porteur 1 pourrait bien entendu être réalisée autrement.

Elle pourrait ainsi être réalisée automatiquement par l'unité de calcul qui traiterait les images acquises par la caméra de manière à y repérer la monture de lunettes et qui piloterait en conséquence la bascule à une hauteur telle que la monture de lunettes se trouve au centre des images acquises par la caméra.

En variante, si le capteur d'images n'est pas monté mobile par rapport au sol, cette opération de positionnement pourra être réalisée en demandant au porteur de s'asseoir sur un tabouret dont la hauteur aura au préalable été réglée.

Une fois cette opération de positionnement du porteur 1 par rapport à la caméra 210 réalisée, le vendeur initie la prise de mesure en appuyant sur un bouton ad hoc affiché sur l'écran tactile 270.

L'unité de pilotage 250 commande alors l'allumage des trois sources de lumière 220, 230, 240 infrarouges, à une intensité nominale.

Bien entendu, l'unité de pilotage pourrait moduler cette intensité, en fonction de l'intensité de la lumière extérieure, pour éviter que cette dernière ne perturbe les mesures. L'utilisation d'une lumière infrarouge permet toutefois de limiter ces perturbations, si bien que l'intensité nominale est généralement suffisante.

L'utilisation de trois sources de lumière 220, 230, 240 distinctes assure alors la présence d'au moins un reflet sur chaque lentille de présentation 150G, 150D de la paire de lunettes 100, pour autant bien sûr que le porteur 1 ne détourne pas complètement la tête.

Dans la variante où le kiosque ne comporterait qu'une source de lumière mobile en hauteur par rapport à la caméra, l'opération de positionnement du porteur en face de la caméra serait suivie d'une opération automatique de positionnement de la source de lumière. Au cours de cette opération, l'unité de calcul ferait varier la hauteur de la source de lumière et traiterait les images acquises par la caméra pour immobiliser la source de lumière à une hauteur telle que les reflets-verres soient centrés sur les lentilles de présentation.

Quoi qu'il en soit, le vendeur demande alors au porteur 1 de tourner la tête 10 de droite à gauche et de gauche à droite, autour de l'axe longitudinal A1 (de manière à faire « non » de la tête).

Il lui indique que le mouvement doit être réalisé lentement (avec une période supérieure à la seconde), avec une amplitude réduite (d'environ 10 degrés).

En variante, on pourrait prévoir de monter la caméra et les sources de lumière mobiles de manière à ce qu'elles pivotent ensemble autour de l'axe longitudinal de la tête du porteur. Ainsi, le porteur n'aurait pas à faire de mouvement de tête.

Puis, lorsque le porteur 1 commence à tourner la tête de la manière demandée, le vendeur appuie sur un bouton de démarrage des mesures affiché sur l'écran tactile 270.

La caméra acquiert alors une pluralité d'images-infrarouges, sur lesquelles apparaissent la tête 10 du porteur 1 et sa paire de lunettes 100.

Les images acquises sont tout d'abord mémorisées dans la mémoire vive de l'unité de calcul 250. Certaines d'entre elles, jugées exploitables, sont alors sélectionnées par l'unité de calcul 250 et mémorisées dans sa mémoire morte. La sélection des images acquises est réalisée de la manière suivante.

Une image acquise peut présenter plusieurs ' reflets-verres (au maximum 6), chaque reflet-verre 160G, 160D, 161G, 161D est noté individuellement sur différents critères, notamment :

- de forme (largeur, hauteur, ratio largeur/hauteur), et
- d'intensité (luminance).

Chaque couple de reflets (générés par une même source de lumière et respectivement réfléchis par les deux lentilles de présentation) est par ailleurs noté selon d'autres critères, notamment :

- de distance entre les reflets,
- d'horizontalité par rapport à l'axe horizontal des cadres boxing, et
- de comparaison des surfaces des reflets.

Ici, la note attribuée à chaque critère n'est pas binaire.

On peut par exemple prévoir qu'elle varie continûment entre 0 et 1 de telle sorte que :

- elle soit égale à 1 si le critère est compris entre 90% et 110% de sa valeur nominale (prédéterminée et mémorisée dans la mémoire morte de l'unité de calcul 250),
- elle soit égale à 0 si le critère est supérieur à 120% ou inférieur à 70% de sa valeur nominale, et
- elle varie linéairement entre 70% et 90% et entre 110% et 120%.

Les critères s'appliquant aux reflets-verres peuvent également s'appliquer mutatis mutandis aux reflets cornéens 162G, 162D. Un critère supplémentaire s'appliquant aux reflets cornéens sera la distance séparant chaque reflet cornéen de l'axe remarquable A3.

Alors, une image a d'autant plus de chance d'être sélectionnée que le produit des notes attribuées aux différents critères précités est important.

On peut par exemple prévoir que les images sélectionnées sont celles pour lesquelles le produit des notes est supérieur à un seuil prédéterminé.

En variante, on peut prévoir que les images sélectionnées sont celles pour lesquelles les produits des notes sont les plus élevés.

On peut par ailleurs prévoir qu'une image est automatiquement rejetée si l'une des notes qui lui a été attribuée est inférieure à un autre seuil prédéterminé.

Ainsi, si sur une image acquise, le porteur 1 a tourné la tête d'un angle de lacet $\alpha$ trop important et que les reflets-verres sont sortis du contour des lentilles de présentation 150G, 150D, cette image est automatiquement rejetée.

Par ailleurs, si sur une image acquise, le porteur 1 a incliné la tête d'un angle de roulis $\beta$ important et que les reflets-verres générés par une même source de lumière son décalés en hauteur sur les deux lentilles de présentation, cette image pourra être rejetée si ce décalage est vraiment très important, ou sélectionnée si les notes attribuées aux autres critères sont élevées.

L'unité de calcul 250 obtient ainsi un nombre restreint d'images-infrarouges exploitables pour la suite du procédé.

Pour la clarté de cet exposé, on considérera alors que l'unité de calcul 250 n'a sélectionné que trois images-infrarouges 301, 302, 303 représentées sur les figures 5 à 7.

Préférentiellement, l'unité de calcul arrêtera l'acquisition d'images lorsqu'elle aura sélectionnée au moins 10 images différentes.

L'unité de calcul 250 commande ensuite le basculement du filtre infrarouge de la caméra 210 pour mémoriser une image-visible du visage du porteur 1.

Elle commande simultanément l'extinction des sources de lumières 220, 230, 240 infrarouges, et l'affichage d'un message sur l'écran tactile 270 indiquant au porteur qu'il peut arrêter de tourner la tête.

Ce message est donc affiché après que l'unité de calcul 250 a sélectionné un nombre prédéterminé d'images, ici égal à 10.

En variante, on pourrait prévoir d'afficher ce message après une durée prédéterminée, par exemple égale à 10 secondes, à l'issue de laquelle on estime pouvoir obtenir un nombre suffisant d'images exploitables. Les images pourront alors être sélectionnées dans un second temps, après que l'ensemble des images ont été acquises.

Une fois les images 301, 302, 303 sélectionnées, l'unité de calcul 250 procède au calcul de l'angle de lacet $\alpha$ de la tête 10 du porteur 1 sur chacune de ces images.

Le calcul de cet angle de lacet $\alpha$ est opéré en localisant sur chaque image 301, 302, 303 les reflets-verres 160D, 160G, 161D, 161G par rapport au plan sagittal P2 de la tête du porteur 1.

Ici, cet angle de lacet $\alpha$ est plus précisément déterminé en localisant les reflets-verres 160D, 160G, 161D, 161G par rapport à l'axe remarquable A3 de la monture de lunettes (dont la position correspond en effet sensiblement à l'intersection du plan sagittal P2 avec le plan moyen P1 de la monture de lunettes 110).

La position de l'axe remarquable A3 de la monture de lunettes 110 est obtenu en 3 étapes consistant à :

- repérer sur chaque image 301, 302, 303 la monture de lunettes 110, et notamment les cercles 111D, 111G de cette monture de lunettes,
- positionner sur chaque image 301, 302, 303 les cadres boxing 151G, 151D, et
- déterminer la position de l'axe passant entre ces deux cadres boxing, qui correspond en pratique à l'axe remarquable A3.

L'utilisation d'une lumière infrarouge permet ici de faciliter le repérage de la monture de lunettes 110, dont le contour se détache bien de celui du visage du porteur 1.

Alors, l'angle de lacet $\alpha$ de la tête10 du porteur 1 sur chaque image 301, 302, 303 est calculé en fonction des distances séparant les reflets-verres 160D, 160G, 161D, 161G de l'axe remarquable A3.

Typiquement, comme le montrent bien les figures 5 à 7, l'unité de calcul 250 détermine sur chaque image 301, 302, 303 les distances $XD_{301}$, $XG_{301}$, $XD_{302}$, $XG_{302}$, $XD_{303}$, $XG_{303}$ séparant l'axe remarquable A3 des centres des deux reflets-verres 160G, 160D les plus gros (les reflets-verres générés par les sources secondaires n'étant exploités que si les reflets-verres générés par la source principale n'apparaissent par sur les images acquises).

L'angle de lacet $\alpha_i$ de la tête 10 du porteur 1 sur chaque image 301, 302, 303 est alors déterminé de la manière suivante :

$\alpha_i = k. (XG_i - XD_i) / (XG_i + XD_i)$, avec i allant de 301 à 303.

Le coefficient k est alors une constante relative au galbe de la monture de lunettes.

Ce coefficient k pourra être formé par une constante prédéterminée et invariable, mémorisée dans la mémoire morte de l'unité de calcul 250. Elle sera donc toujours la même quelle que soit la monture de lunettes sélectionnée par le porteur. Ce coefficient k pourra alors être déterminé expérimentalement sur la base d'un panel représentatif de montures de lunettes, et ainsi choisi environ égal à 40°.

En variante, ce coefficient k pourra être formé par une constante prédéterminée associée à la monture de lunettes sélectionnée par le client. Dans cette variante, ce coefficient k sera alors déterminé expérimentalement pour chaque référence de monture de lunettes, puis mémorisé dans la base de données de manière à être accessible lors du calcul de l'angle de lacet $\alpha_i$ de la tête 10 du porteur 1.

La détermination de cet angle de lacet pourrait être réalisée autrement.

A titre d'exemple, on pourrait prévoir d'équiper la monture de lunettes d'un système de repérage comportant des motifs géométriques facilement repérables et écartés d'une distance réelle connue, et de déterminer l'angle de lacet en fonction de la distance séparant ces motifs géométriques sur l'image acquise (préalablement mise à l'échelle 1 : 1). Un tel procédé est présenté en détail dans le document WO 2008/132356.

A ce stade, l'unité de calcul 250 a en mémoire les angles de lacet $\alpha_{301}$, $\alpha_{302}$, $\alpha_{303}$ de la tête10 du porteur 1 sur chaque image 301, 302, 303 sélectionnée.

Elle procède alors à la détermination des demi-écarts pupillaires EG, ED du porteur 1, en fonction des positions des reflets cornéens 162G, 162D du porteur 1 sur au moins une des images 301, 302, 303 sélectionnées.

Elle peut pour cela procéder de différentes manières. Deux méthodes différentes seront ici exposées.

La première méthode consiste à élire une image 302 parmi les images 301, 302, 303 sélectionnées, à savoir celle pour laquelle l'angle de lacet $\alpha_{302}$ calculé est le plus faible, puis à considérer que cette image élue 302, la tête 10 du porteur 1 est de face. Les demi-écarts pupillaires EG, ED pourront alors être directement lus sur cette image 302.

Cette méthode nécessite toutefois d'avoir acquis et sélectionné un nombre suffisant d'images 301, 302, 303, de manière que parmi ces images, l'une d'entre elles représente effectivement la tête du porteur 1 vue sensiblement de face.

Cette méthode est alors plus précisément mise en œuvre de la manière suivante.

L'unité de calcul 250 procède au repérage sur cette image élue 302 des reflets cornéens 162G, 162D, qui sont excentrés par rapport aux reflets-verres.

Puis, l'unité de calcul 258 détermine les distances $EG_{302}$, $ED_{302}$ séparant l'axe remarquable A3 de chacun de ces reflets cornéens 162G, 162D.

Ces distances $EG_{302}$, $ED_{302}$ sont ensuite mises à l'échelle, ce qui permet à l'unité de calcul 250 d'obtenir les demi-écarts pupillaires EG, ED du porteur 1.

La seconde méthode consiste à considérer toutes les images 301, 302, 303 sélectionnées, puis, par une méthode statistique, à évaluer les demi-écarts pupillaires EG, ED du porteur 1.

Cette méthode statistique permet non seulement d'évaluer les demi-écarts pupillaires EG, ED du porteur 1, mais également d'évaluer les distances œil-lunettes VG, VD séparant le plan moyen P1 de la monture de lunettes 110 et les centres de rotation OG, OD des yeux 20G, 20D du porteur 1.

Cette méthode statistique est alors mise en œuvre sur l'un des deux yeux 20G, 20D du porteur 1.

Ici, comme le montre la figure 8, elle sera mise en œuvre sur l'œil gauche 20G du porteur 1 afin de déterminer son demi-écart pupillaire gauche EG et sa distance œil-lunettes gauche VG.

Globalement la méthode statistique part du constat que, grâce au reflet cornéen gauche 162G apparaissant sur chaque image 301, 302, 303, on sait sur quel axe A9 (voir figure 8) se trouve le centre de rotation OG de l'œil gauche 20G du porteur 1, et qu'il reste donc seulement à déterminer la position du centre de rotation OG sur cet axe A9.

Cette méthode statistique va alors consister :

- à simuler, sur chaque image 301, 302, 303, une pluralité de distances oeil-lunettes VG1, VG2, VG3 (ici au nombre de 3 pour la clarté de l'exposé et des dessins),

- à déterminer le demi-écart pupillaire gauche correspondant à chacune de ces distances simulées (appelé « demi-écart pupillaire gauche simulé $EG1_{301}$, $EG2_{301}$, $EG3_{301}$, $EG1_{302}$, $EG2_{302}$, $EG3_{302}$, $EG1_{303}$, $EG2_{303}$, $EG3_{303}$») , puis, en fonction de l'ensemble de ces demi-écarts pupillaires gauches simulés,
- à déterminer laquelle des distances œil-lunettes simulées VG1, VG2, VG3 est la plus proche de la distance œil-lunette VG réelle du porteur 1.

Plus précisément, l'unité de calcul 250 considère ici trois distances oeil-lunettes simulées VG1, VG2, VG3 prédéterminées et mémorisées dans sa mémoire morte. En variante et de manière préférentielle, elle considérera plutôt un nombre de distances œil-lunettes simulées supérieur à 10.

Alors, pour chacune de ces distances œil lunettes simulées, et pour chacune des images acquises (indice i, variant dans cet exemple de 301 à 303), l'unité de calcul 250 calcule de manière itérative (indice d'itération k variant de 1 à n) :

$$Z1_{i,k} = Zb_i + VG1.\cos(\alpha_i) + EG1_{i,k-1}.\sin(\alpha_i),$$

$$Z2_{i,k} = Zb_i + VG2.\cos(\alpha_i) + EG2_{i,k-1}.\sin(\alpha_i),$$

$$Z3_{i,k} = Zb_i + VG3.\cos(\alpha_i) + EG3_{i,k-1}.\sin(\alpha_i),$$

avec :

- $EG1_{i,0}$, $EG2_{i,0}$, $EG3_{i,0}$ fixé arbitrairement à une valeur prédéterminée, ici choisie égale à 32,5 mm ;
- $Zb_i$ la distance séparant l'axe remarquable A3 et le plan général P4 de l'objectif de la caméra 210 sur chaque image (et qui est facilement déterminable, compte tenu de la largeur de la monture sur l'image considérée par rapport à sa largeur réelle, et de l'angle de lacet $\alpha_i$ de la tête du porteur) ;
- $Z1_{i,k}$, $Z2_{i,k}$, $Z3_{i,k}$ les distances séparant la position simulée du centre de rotation OG de l'œil gauche 20G du porteur 1 et le plan général P4 de l'objectif de la caméra 210.

Si on note :

- $x_{OGi}$ et $y_{OGi}$ : les coordonnées, exprimées en pixel, du centre de rotation OG dans l'image i, par rapport au centre de cette image,
- X,Y,Z : les coordonnées, exprimées en mm, du centre de rotation OG dans le repère de la caméra, et
- K, la taille d'un pixel, exprimée en mm, pour un objet situé à un mètre du plan de la caméra.

Alors, on peut écrire les équations suivantes :

$$X1_{i,k} = Z1_{i,k} . K . x_{OGi}$$

$$Y1_{i,k} = Z1_{i,k} . K . y_{OGi}$$

$$Z1_{i,k} = Z1_{i,k}$$

$$X2_{i,k} = Z2_{i,k} . K . x_{OGi}$$

$$Y2_{i,k} = Z2_{i,k} . K . y_{OGi}$$

$$Z2_{i,k} = Z2_{i,k}$$

$$X3_{i,k} = Z3_{i,k} \cdot K \cdot x_{OGi}$$

$$Y3_{i,k} = Z3_{i,k} \cdot K \cdot y_{OGi}$$

$$Z3_{i,k} = Z3_{i,k}$$

Pour l'image i, le plan sagittal, noté $P2_i$ peut être, par exemple, défini comme le plan qui inclut les trois points suivants :

- $A_i$ : un point situé sur l'axe remarquable A3, correspondant au centre du segment reliant les deux coins supérieurs les plus proches des deux cadres boxing 151G, 151D,
- $B_i$ : un point situé sur l'axe remarquable A3, correspondant au centre du segment reliant les deux coins inférieurs les plus proches des deux cadres boxing 151G, 151D,
- $C_i$ : un point situé sur un axe qui passe par le point $A_i$, qui est perpendiculaire au plan moyen P1 de la monture de lunettes 110, et dont les coordonnées dans le repère de la monture sont telles que le vecteur $A_iC_i$ a pour coordonnées $(x_c, y_c, z_c)$ avec $x_c = 0$ et $y_c = 0$.

Si on pose :

- $(x_{Ai}, y_{Ai})$ les coordonnées du point $A_i$ dans l'image i,
- $X_{Ai}, Y_{Ai}, Z_{Ai}$, les coordonnées du point $A_i$ dans le repère de la caméra,
- $(x_{Bi}, y_{Bi})$ les coordonnées du point $A_i$ dans l'image i,
- $X_{Bi}, Y_{Bi}, Z_{Bi}$ les coordonnées du point $B_i$ dans le repère de la caméra,
- $X_{Ci}, Y_{Ci}, Z_{Ci}$ les coordonnées du point $C_i$ dans le repère de la caméra, et
- $M_i$, la matrice de rotation tridimensionnelle décrivant la posture de la monture dans l'espace de la caméra (construite à partir des angles $\alpha$, $\beta$ et $\delta$).

Alors, comme la distance de l'axe remarquable A3 au plan de la caméra P4 est connue, $Z_{Ai}$ et $Z_{Bi}$ sont connus et on peut écrire les équations suivantes :

$$X_{Ai} = Z_{Ai} \cdot K \cdot x_{Ai}$$

$$Y_{Ai} = Y_{Ai} \cdot K \cdot y_{Ai}$$

$$X_{Bi} = Z_{Bi} \cdot K \cdot x_{Bi}$$

$$Y_{Bi} = Y_{Bi} \cdot K \cdot y_{Bi}$$

Et, après inversion de la matrice de rotation M :

$$(X_{Ci}-X_{Ai}) = M^{-1}(0,0) \cdot x_c + M^{-1}(0,1) \cdot y_c + M^{-1}(0,2) \cdot z_c$$

$$(Y_{Ci}-Y_{Ai}) = M^{-1}(1,0) \cdot x_c + M^{-1}(1,1) \cdot y_c + M^{-1}(1,2) \cdot z_c$$

$$(Z_{Ci}-Z_{Ai}) = M^{-1}(2,0) \cdot x_c + M^{-1}(2,1) \cdot y_c + M^{-1}(2,2) \cdot z_c$$

Comme, $x_c$ et $y_c$ sont égaux à zéro par construction, on en déduit que :

$$X_{Ci} = X_{Ai} + M^{-1}(0,2) \cdot z_c$$

$$Y_{Ci} = Y_{Ai} + M^{-1}(1,2) \cdot z_c$$

$$Z_{Ci} = Z_{Ai} + M^{-1}(2,2) \cdot z_c$$

Les coordonnées des 3 points du plan P2$_i$ étant connues, celui-ci est parfaitement défini, et il est donc possible de calculer selon la méthode classique la distance d'un point quelconque au plan P2$_i$.

On peut donc en particulier calculer EG1$_{i,k}$, EG2$_{i,k}$, EG3$_{i,k}$, avec

- EG1$_{i,k}$ : la distance séparant le centre de rotation OG du plan P2$_i$, en considérant la distance œil-lunettes VG1 ;
- EG2$_{i,k}$ : la distance séparant le centre de rotation OG du plan P2$_i$, en considérant la distance œil-lunettes VG2 ;
- EG3$_{i,k}$ : la distance séparant le centre de rotation OG du plan P2$_i$, en considérant la distance oeil-lunettes VG3.

Ces valeurs peuvent alors être réinjectées dans les formules initiales pour continuer l'itération.

Le calcul itératif s'arrête soit après un nombre prédéterminé d'itérations (par exemple pour k = 3), soit lorsque pour deux itérations successives, les valeurs de EG1$_{i,k}$ et EG1$_{i,k+1}$ , celles de EG2$_{i,k}$ et EG2$_{i,k+1}$ et celles de EG3$_{i,k}$ et EG3$_{i,k+1}$ sont proches (à savoir ici inférieures à 0,1 mm).

Les valeurs de EG1$_{i,k}$, EG2$_{i,k}$, EG3$_{i,k}$ obtenues sont alors considérées comme étant les demi-écarts pupillaires simulés EG1$_i$, EG2$_i$, EG3$_i$.

Une fois l'ensemble des demi-écarts pupillaires simulés EG1$_i$, EG2$_i$, EG3$_i$ calculés, l'unité de calcul 250 sélectionne, parmi les distances œil-lunettes simulées VG1, VG2, VG3, celle qui est la plus proche de la valeur réelle VG.

Pour cela, l'unité de calcul 250 détermine, pour chaque distance œil-lunettes simulée VG1, VG2, VG3, l'écart-type $\sigma_1$, $\sigma_2$, $\sigma_3$ des demi-écarts pupillaires simulés EG1$_i$, EG2$_i$, EG3$_i$ associés.

A titre d'exemple, l'écart-type $\sigma_1$ associé à la première distance oeil-lunettes simulée VG1 est égal à l'écart-type des demi-écarts pupillaires simulés EG1$_{303}$, EG1$_{302}$, EG1$_{303}$.

Alors, la distance œil-lunettes simulée VG2 sélectionnée est celle pour laquelle l'écart-type $\sigma_2$ est le plus faible.

L'unité de calcul 250 considère alors que cette distance œil-lunettes simulée VG2, une fois mise à l'échelle, est égale à la distance œil-lunettes VG réelle du porteur 1. Elle mémorise donc sa valeur.

L'unité de calcul 250 calcule ensuite la moyenne des demi-écarts pupillaires simulés EG2$_{301}$, EG2$_{302}$, EG2$_{303}$ associés à cette valeur simulée sélectionnée VG2.

Elle considère ensuite que cette moyenne, une fois mise à l'échelle, est égale au demi-écart pupillaire gauche EG du porteur 1. Elle mémorise donc sa valeur.

La détermination de la distance œil-lunettes droite VD et du demi-écart pupillaire droit ED du porteur 1 peut alors être réalisée de la même manière que pour les distances œil-lunettes gauche VG et demi-écart pupillaire gauche EG du porteur 1.

Au cours d'une opération suivante, l'unité de calcul 250 procède au calcul des hauteurs pupillaires HG, HD du porteur 1 (voir figure 1).

Encore une fois, l'unité de calcul 250 peut opérer de diverses manières.

Ici, on considérera que le tangage de la tête 10 du porteur 1 a peu d'incidence sur la précision des hauteurs pupillaires HG, HD que l'on peut mesurer sur les images 301, 302, 303.

Par conséquent, pour déterminer ces hauteurs pupillaires, l'unité de calcul 250 sélectionne l'une quelconque des images 301, 302, 303 (par exemple celle pour laquelle l'angle de lacet $\alpha_{302}$ est le plus faible) puis détermine sur cette image 302 (figure 6) les distances HG$_{302}$, HD$_{302}$ séparant chacun des reflets cornéens 162G, 162D du bord inférieur du cadre boxing 151G, 151D correspondant.

Ces distances HG$_{302}$, HD$_{302}$, une fois mises à l'échelle, sont alors mémorisées par l'unité de calcul 250 comme étant les hauteurs pupillaires HG, HD.

En variante, l'unité de calcul pourra mesurer ces distances HG$_i$, HD$_i$ sur chacune des images 301, 302, 303, moyenner ces distances et les mettre à l'échelle afin d'obtenir les hauteurs pupillaires du porteur.

Selon une autre variante, on pourra procéder au calcul des hauteurs pupillaires en suivant une méthode homologue de celle utilisée pour déterminer les demi-écarts pupillaires du porteur. Cette méthode consistera par exemple à acquérir différentes images du porteur faisant « oui » de la tête, à déterminer les angles de tangage de la tête sur chacune de ces images, et à mesurer les hauteurs pupillaires sur l'image pour laquelle l'angle de tangage est le plus faible.

L'opération suivante consiste pour l'unité de calcul 250 à déterminer la propension du porteur 1 à détourner le regard en bougeant plutôt la tête 10 ou les yeux 20G, 20D.

Le vendeur explique alors au porteur 1 qu'il doit regarder la première cible supplémentaire 320, puis, dès que celle-ci s'éteint et que la seconde cible supplémentaire 330 s'illumine, qu'il doit détourner rapidement le regard vers cette seconde cible supplémentaire 330.

Dès que le porteur 1 est prêt, le vendeur initie une nouvelle prise de mesures en appuyant sur un bouton ad hoc affiché sur l'écran tactile 270.

L'unité de calcul 250 commande alors l'allumage de la première cible supplémentaire 320 seule, pendant environ 5 secondes, puis l'extinction de la première cible supplémentaire 320 et l'allumage simultané de la seconde cible supplémentaire 330. Au moment de l'extinction de la première cible supplémentaire 320, l'unité de calcul 250 commande l'acquisition et la mémorisation d'une pluralité d'images (par exemple 10) au cours d'une séquence d'environ 1 seconde.

Les images acquises sont alors traitées par l'unité de calcul 250 pour déterminer sur chacune d'entre elles l'angle de lacet $\alpha_i$ de la tête 10 du porteur 1.

Si, sur les 10 images acquises, cet angle varie peu et/ou lentement, l'unité de calcul 250 détermine que le porteur a une propension à détourner le regard en bougeant plutôt les yeux 20G, 20D.

Au contraire, si, sur les 10 images acquises, cet angle varie beaucoup et/ou rapidement, l'unité de calcul 250 détermine que le porteur a une propension à détourner le regard en bougeant plutôt la tête 10.

En variante, l'unité de calcul pourrait procéder autrement.

A titre d'exemple, elle pourrait procéder plus finement, en déterminant également sur chaque image acquise les distances $EG_i$, $ED_i$ séparant les reflets cornéens 162D, 162G de l'axe remarquable A3.

Elle pourrait alors comparer les vitesses et/ou amplitudes de variation de l'angle de lacet $\alpha_i$ avec les vitesses et/ou amplitudes de variation des distances $EG_i$, $ED_i$. Elle pourrait ainsi en déduire si le porteur a une propension à tourner plus rapidement les yeux ou la tête. Elle pourrait même en déduire un coefficient fin, quantifiant la propension du porteur à détourner le regard en bougeant plutôt la tête ou les yeux.

A l'issue de ces différentes opérations, l'unité de calcul 250 a acquis :

- une image scannée des prescriptions du porteur,
- la référence de la monture de lunettes sélectionnée par le porteur 1,
- les demi-écarts pupillaires EG, ED du porteur 1,
- les hauteurs pupillaires HG, HD du porteur 1,
- une image-visible du visage du porteur 1,
- les distances VG2, VD2 séparant les centres de rotation des yeux du porteur 1 et le plan moyen P1 de la monture de lunettes 110, et
- un coefficient quantifiant la propension du porteur 1 à détourner le regard en bougeant plutôt la tête 10 ou les yeux 20G, 20D.

Elle commande alors l'affichage de ces différentes informations sur l'écran tactile 270 pour vérification par le vendeur.

Après validation par le vendeur, l'unité de calcul 250 commande l'impression de ces informations sur une fiche récapitulative. Elle communique par ailleurs ces informations au centre de fabrication des lentilles ophtalmiques.

On pourra éventuellement prévoir que l'unité de calcul 250 communique en outre au centre de fabrication des lentilles ophtalmiques l'ensemble des images acquises.

Alors, le centre de traitement pourra éventuellement réaliser une opération de vérification de toutes les données calculées par l'unité de calcul 250.

Ces données pourront notamment être vérifiées lorsque le logiciel installé dans l'unité de calcul 250 n'aura pas été mis à jour.

Elles pourront également être vérifiées lorsque la monture sélectionnée sera de type « sans cercle » (ou « percée »). En effet, les bords des lentilles de présentation de ces montures sont généralement peu visibles sur les images acquises, ce qui peut générer des erreurs.

Cette opération de vérification pourra être mise en oeuvre automatiquement (notamment dans le cas où le logiciel n'est pas à jour), ou manuellement, par un technicien spécialisé qui pourra notamment vérifier que les pupilles et les bords des lentilles de présentation ont bien été repérés.

On pourra en particulier prévoir que le dispositif de mesure se présente, non pas sous la forme d'un kiosque logeant l'unité de calcul, le capteur d'images et les sources de lumière, mais plutôt sous une forme de taille réduite et portable. On pourra ainsi prévoir que l'unité de calcul soit formée par un ordinateur portable dans lequel sera installé un logiciel ad hoc, et dont la caméra (ou « web-cam ») fera office de caméra dans le domaine du visible. Dans cette variante, la caméra infrarouge et la source de lumière seront alors agencées sur un boîtier de petite taille, muni d'une pince de fixation à l'écran de l'ordinateur portable et d'un câble de branchement à l'ordinateur portable. Cette solution sera peu onéreuse et facilement transportable.

Selon une autre variante de réalisation de l'invention, on pourra prévoir que l'unité de calcul se contente d'enregistrer des images et de les communiquer au centre de fabrication de lentilles ophtalmiques, auquel cas ces images seront traitées dans ce centre de fabrication.

## Revendications

1. Procédé de détermination d'au moins une caractéristique d'optimisation d'implantation d'une paire de lunettes (100) sur le visage d'un porteur (1), ladite caractéristique d'optimisation comprenant une caractéristique géométrico-morphologique (EG, ED, HD, HG), une caractéristique de posture ($\alpha$) et une caractéristique comportementale, ladite paire de lunettes (100) comprenant une monture (110) et deux lentilles (150G, 150D), le procédé comportant des étapes :

   a) d'éclairage d'au moins une partie de la tête (10) du porteur (1) à l'aide d'une source de lumière infrarouge (220, 230, 240),
   b) d'acquisition par un capteur d'images infrarouges (210) d'une image (301, 302, 303) de la tête (10) du porteur (1) éclairée par la source de lumière infrarouge (220, 230, 240), sur laquelle apparaissent la paire de lunettes (100) et les yeux du porteur (1),
   c) de localisation sur l'image (301, 302, 303) acquise à l'étape b) des reflets-verres (160G, 160D, 161G, 161D) de la source de lumière infrarouge (220, 230, 240) réfléchis par les deux lentilles (150G, 150D) de la paire de lunettes (100) et d'au moins un reflet cornéen (162D, 162G) de la source de lumière infrarouge (220, 230, 240) réfléchi par l'un des deux yeux du porteur (1), et
   d) de déduction par une unité de calcul (250) de :

      - ladite caractéristique de posture ($\alpha$) en fonction des positions des reflets-verres (160G, 160D, 161G, 161D) sur ladite image (301, 302, 303),
      de ladite caractéristique comportementale en fonction de ladite caractéristique de posture ($\alpha$) et de
      - ladite caractéristique géométrico-morphologique (EG, ED, HD, HG) en fonction de la position dudit reflet cornéen (162D, 162G) sur ladite image (301, 302, 303).

2. Procédé de détermination selon la revendication précédente, dans lequel, à l'étape c), on localise sur l'image (301, 302, 303) acquise à l'étape b) les reflets cornéens (162D, 162G) de la source de lumière infrarouge (220, 230, 240) réfléchis par les deux yeux du porteur (1).

3. Procédé de détermination selon l'une des revendications précédentes, dans lequel, à l'étape b), ladite image (301, 302, 303) est acquise dans le domaine du proche infrarouge, avec une longueur d'onde comprise entre 780 et 1400 nm.

4. Procédé de détermination selon l'une des revendications précédentes, dans lequel ladite caractéristique de posture comporte un angle de lacet ($\alpha$), qui sépare le plan sagittal (P2) de la tête (10) du porteur (1) et l'axe optique (A2) du capteur d'images infrarouges (210).

5. Procédé de détermination selon l'une des revendications précédentes, dans lequel ladite caractéristique géométrico-morphologique comporte les demi-écarts pupillaires (EG, ED) du porteur (1).

6. Procédé de détermination selon l'une des revendications précédentes, dans lequel ladite caractéristique géométrico-morphologique comporte les hauteurs pupillaires (HG, HD) du porteur.

7. Procédé de détermination selon l'une des revendications précédentes, dans lequel à l'étape b), le capteur d'images infrarouges (210) acquiert au moins deux images (301, 302, 303) de la tête (10) du porteur (1), à l'étape c), on localise lesdits reflets (160G, 160D, 161G, 161D) sur chaque image (301, 302, 303) acquise à l'étape b), et, à l'étape d), ladite caractéristique comportementale comporte un coefficient quantifiant la propension du porteur à détourner le regard en bougeant plutôt la tête ou les yeux.

8. Procédé de détermination selon l'une des revendications précédentes, dans lequel ladite caractéristique géométrico-morphologique comporte les positions des centres de rotation (OG, OD) des yeux du porteur (1).

9. Dispositif de détermination (200) d'une caractéristique géométrico-morphologique (EG, ED, HD, HG), d'une carac-

téristique de posture ($\alpha$) et d'une caractéristique comportementale d'un individu (1), ce dispositif comportant:

- au moins une source de lumière infrarouge (220, 230, 240) adaptée à éclairer au moins une partie de la tête (10) dudit individu (1),
- un capteur d'images infrarouges (210) adapté à acquérir une image (301, 302, 303) d'au moins une partie de la tête (10) dudit individu (1) éclairée par la source de lumière infrarouge (220, 230, 240), et
- une unité de calcul (250) adaptée à localiser sur ladite image (301, 302, 303) des reflets cornéens ( 162D, 162G) de la source de lumière infrarouge (220, 230, 240) réfléchis par les pupilles dudit individu (1) et de reflets-verres (160G, 160L, 161G, 161L) de la source de lumière infrarouge réfléchis par les deux lentilles (150D, 150G) d'une paire de lunettes (100) portée par ledit individu (1), et à déduire :

- ladite caractéristique de posture ($\alpha$) en fonction des positions des reflets-verres (160G, 160D, 161G, 161D) réfléchis par les deux lentilles (150D, 150G),
de ladite caractéristique comportementale en fonction de ladite caractéristique de posture ($\alpha$) et de
- la caractéristique géométrico-morphologique (EG, ED, HD, HG) en fonction de la position desdit reflets cornéens (162D, 162G) sur ladite image (301, 302, 303).

**10.** Dispositif de détermination selon la revendication 9, dans lequel l'unité de calcul est formée par une unité centrale d'un ordinateur et dans lequel la source de lumière infrarouge et le capteur d'images infrarouges sont dissociés de l'unité de calcul et sont équipés de moyens de communication adaptés à communiquer avec l'unité centrale de l'ordinateur.

**11.** Dispositif de détermination (200) selon la revendication 9, comprenant un bâti (201) auquel sont solidarisés la source de lumière infrarouge (220, 230, 240), le capteur d'images infrarouges (210) et l'unité de calcul (250).

**12.** Dispositif de détermination (200) selon la revendication précédente, dans lequel le bâti (201) comprend un châssis (202) et une coulisse (205) qui est montée mobile sur le châssis (202) et qui supporte la source de lumière infrarouge (220, 230, 240) et/ou le capteur d'images infrarouges (210).

**13.** Dispositif de détermination (200) selon la revendication précédente, dans lequel il est prévu des moyens de motorisation de la mobilité de la coulisse (205), et dans lequel ladite unité de calcul (250) est adaptée à piloter automatiquement lesdits moyens de motorisation dans une position telle que le visage dudit individu (1) soit visible par le capteur d'images infrarouges (210).

**14.** Dispositif de détermination selon l'une des revendications 9 à 13, dans lequel il est prévu une unique source de lumière montée mobile par rapport audit capteur d'image ou au moins deux sources de lumière distantes l'une de l'autre pour générer deux reflets distincts sur ladite image.

**15.** Dispositif de détermination (200) selon l'une des revendications 9 à 14, dans lequel chaque source de lumière infrarouge (220, 230, 240) est composée d'au moins une diode électroluminescente.

**16.** Dispositif de détermination (200) selon l'une des revendications 9 à 15, dans lequel le capteur d'images infrarouges (210) est également adapté à acquérir des images dans le domaine visible.

**17.** Dispositif de détermination selon l'une des revendications 9 à 15, dans lequel il est prévu un capteur d'images visibles, distinct du capteur d'images infrarouges, qui est adapté à acquérir une image du porteur dans le domaine visible.

**18.** Dispositif de détermination (200) selon l'une des revendications 16 et 17, dans lequel il est prévu des moyens d'affichage (270) et/ou d'impression (280) des images visibles acquises.

**19.** Dispositif de détermination (200) selon l'une des revendications 9 à 18, dans lequel il est prévu des moyens d'acquisition (290) d'au moins une dimension de la paire de lunettes (100).

**Patentansprüche**

**1.** Verfahren zum Bestimmen wenigstens einer Anordnungsoptimierungscharakteristik einer Brille (100) im Gesicht eines Trägers (1), wobei die Optimierungscharakteristik eine geometrisch-morphologische Charakteristik (EG, ED, HD, HG), eine Haltungscharakteristik ($\alpha$) und eine Verhaltenscharakteristik umfasst, wobei die Brille (100) ein Gestell (110) und zwei Gläser (150G, 150D) umfasst, wobei das Verfahren die folgenden Schritte umfasst:

a) Beleuchten wenigstens eines Teils des Kopfes (10) des Trägers (1) mit Hilfe wenigstens einer Infrarotlichtquelle (220, 230, 240),

b) Erfassen durch einen Infrarotbildsensor (210) eines Bildes (301, 302, 303) des mit der Infrarotlichtquelle (220, 230, 240) beleuchteten Kopfes (10) des Trägers (1), in dem die Brille (100) und die Augen des Trägers (1) sichtbar sind,

c) Lokalisieren in dem im Schritt b) erfassten Bild (301, 302, 303) von Glasreflexen (160G, 160D, 161G, 161D) der Infrarotlichtquelle (220, 230, 240), die von den beiden Gläsern (150G, 150D) der Brille (100) reflektiert werden, und wenigstens eines Hornhautreflexes (162D, 162G) der Infrarotlichtquelle (220, 230, 240), der von einem der beiden Augen des Trägers (1) reflektiert wird, und

d) Ableiten durch eine Recheneinheit (250)

- der Haltungscharakteristik ($\alpha$) als Funktion der Positionen der Glasreflexe (160G, 160D, 161G, 161D) auf dem Bild (301, 302, 303),
- der Verhaltenscharakteristik als Funktion der Haltungscharakteristik ($\alpha$) und
- der geometrisch-morphologischen Charakteristik (EG, ED, HD, HG) als Funktion der Position des Hornhautreflexes (162D, 162G) auf dem Bild (301, 302, 303) trennen.

2. Bestimmungsverfahren nach dem vorhergehenden Anspruch, wobei im Schritt c) in dem im Schritt b) erfassten Bild (301, 302, 303) die Hornhautreflexe (162D, 162G) der Infrarotlichtquelle (220, 230, 240), die von den beiden Augen des Trägers (1) reflektiert werden, lokalisiert werden.

3. Bestimmungsverfahren nach einem der vorhergehenden Ansprüche, wobei im Schritt b) das Bild (301, 302, 303) im nahen Infrarotbereich bei einer Wellenlänge im Bereich von 780 bis 1400 nm erfasst wird.

4. Bestimmungsverfahren nach einem der vorhergehenden Ansprüche, wobei die Haltungscharakteristik einen Gierwinkel ($\alpha$) enthält, um den sich die Sagittalebene (P2) des Kopfes (10) des Trägers (1) von der optischen Achse (A2) des Infrarotbildsensors (210) unterscheidet.

5. Bestimmungsverfahren nach einem der vorhergehenden Ansprüche, wobei die geometrisch-morphologische Charakteristik die Pupillen-Halbabstände (EG, ED) des Trägers (1) enthält.

6. Bestimmungsverfahren nach einem der vorhergehenden Ansprüche, wobei die geometrisch-morphologische Charakteristik die Pupillenhöhen (HG, HD) des Trägers enthält.

7. Bestimmungsverfahren nach einem der vorhergehenden Ansprüche, wobei im Schritt b) der Infrarotbildsensor (210) wenigstens zwei Bilder (301, 302, 303) des Kopfes (10) des Trägers (1) erfasst, im Schritt c) die Reflexe (160G, 160D, 161G, 161D) in jedem im Schritt b) erfassten Bild (301, 302, 303) lokalisiert werden und im Schritt d) die Verhaltenscharakteristik einen Koeffizienten enthält, der die Neigung des Trägers, den Blick lieber durch Bewegen entweder des Kopfes oder aber der Augen abzuwenden, quantifiziert.

8. Bestimmungsverfahren nach einem der vorhergehenden Ansprüche, wobei die geometrisch-morphologische Charakteristik die Positionen der Drehzentren (OG, OD) der Augen des Trägers (1) enthält.

9. Vorrichtung (200) für die Bestimmung wenigstens einer geometrisch-morphologischen Charakteristik (EG, ED, HD, HG), einer Haltungscharakteristik ($\alpha$) und einer Verhaltenscharakteristik einer Person (1), wobei diese Vorrichtung Folgendes umfasst:

- wenigstens eine Infrarotlichtquelle (220, 230, 240), die dafür ausgelegt ist, wenigstens einen Teil des Kopfes (10) der Person (1) zu beleuchten,
- einen Infrarotbildsensor (210), der dafür ausgelegt ist, ein Bild (301, 302, 303) wenigstens eines durch die Infrarotlichtquelle (220, 230, 240) beleuchteten Teils des Kopfes (10) der Person (1) zu erfassen, und
- eine Recheneinheit (250), die dafür ausgelegt ist, auf dem Bild (301, 302, 303) Hornhautreflexe (162D, 162G) der Infrarotlichtquelle (220, 230, 240), die von den Pupillen der Person (1) reflektiert werden, und Glasreflexe (160G, 160L, 161G, 161L) der Infrarotlichtquelle, die von den beiden Gläsern (150D, 150G) einer von der Person (1) getragenen Brille (100) reflektiert werden, zu lokalisieren und Folgendes abzuleiten:

- die Haltungscharakteristik ($\alpha$) als Funktion der Positionen von Glasreflexen (160G, 160D, 161G, 161D), die von den beiden Gläsern (150D, 150G) reflektiert werden,
- die Verhaltenscharakteristik als Funktion der Haltungscharakteristik ($\alpha$) und
- die geometrisch-morphologische Charakteristik (EG, ED, HD, HG) als Funktion der Position der Hornhautreflexe (162D, 162G) auf dem Bild (301, 302, 303).

10. Bestimmungsvorrichtung nach Anspruch 9, wobei die Recheneinheit durch eine Zentraleinheit gebildet ist und wobei die Infrarotlichtquelle und der Infrarotbildsensor getrennt von der Recheneinheit vorgesehen sind und mit Kommunikationsmitteln ausgerüstet sind, die dafür ausgelegt sind, mit der Zentraleinheit zu kommunizieren.

11. Bestimmungsvorrichtung (200) nach Anspruch 9, die ein Gestell (201) umfasst, an dem die Infrarotlichtquelle (220, 230, 240), der Infrarotbildsensor (210) und die Recheneinheit (250) fest angebracht sind.

12. Bestimmungsvorrichtung (200) nach dem vorhergehenden Anspruch, wobei das Gestell (201) einen Rahmen (202) und einen Gleiter (205), der an dem Rahmen (202) beweglich montiert ist und die Infrarotlichtquelle (220, 230, 240) und/oder den Infrarotbildsensor (210) trägt, umfasst.

13. Bestimmungsvorrichtung (200) nach dem vorhergehenden Anspruch, wobei Mittel für die kraftbetriebene Bewegung des Gleiters (205) vorgesehen sind und wobei die Recheneinheit (250) dafür ausgelegt ist, die Kraftantriebsmittel automatisch in eine Position zu steuern, derart, dass das Gesicht der Person (1) für den Infrarotbildsensor (210) sichtbar ist.

14. Bestimmungsvorrichtung nach einem der Ansprüche 9 bis 13, wobei eine einzige Lichtquelle, die in Bezug auf den Bildsensor beweglich montiert ist, oder wenigstens zwei voneinander beabstandete Lichtquellen, um auf dem Bild zwei verschiedene Reflexe zu erzeugen, vorgesehen sind.

15. Bestimmungsvorrichtung (200) nach einem der Ansprüche 9 bis 14, wobei jede Infrarotlichtquelle (220, 230, 240) aus wenigstens einer Elektrolumineszenzdiode gebildet ist.

16. Bestimmungsvorrichtung (200) nach einem der Ansprüche 9 bis 15, wobei der Infrarotbildsensor (210) außerdem dafür ausgelegt ist, Bilder im sichtbaren Bereich zu erfassen.

17. Bestimmungsvorrichtung nach einem der Ansprüche 9 bis 15, wobei ein Sensor für sichtbare Bilder vorgesehen ist, der von dem Infrarotbildsensor verschieden ist und dafür ausgelegt ist, ein Bild des Trägers im sichtbaren Bereich zu erfassen.

18. Bestimmungsvorrichtung (200) nach einem der Ansprüche 16 und 17, wobei Mittel (270) zum Anzeigen und/oder Mittel (280) zum Drucken erfasster sichtbarer Bilder vorgesehen sind.

19. Bestimmungsvorrichtung (200) nach einem der Ansprüche 9 bis 18, wobei Mittel (290) zum Erfassen wenigstens einer Abmessung der Brille (100) vorgesehen sind.

## Claims

1. Method for determining at least one characteristic for optimizing a wear position of a pair of spectacles (100) on the face of a wearer (1), said optimizing characteristic comprising a geometrico-morphological characteristic (EG, ED, HD, HG), a postural characteristic ($\alpha$) and a behavioural characteristic, said pair of spectacles (100) comprising a frame (110) and two lenses (150G, 150D), the method comprising steps of:

   a) illuminating at least part of the head (10) of the wearer (1) using an infrared light source (220, 230, 240);
   b) acquiring, via an infrared image sensor (210), an image (301, 302, 303) of the head (10) of the wearer (1) illuminated by the infrared light source (220, 230, 240), in which image the pair of spectacles (100) and the eyes of the wearer (1) appear;
   c) locating, in the image (301, 302, 303) acquired in step b), glass-reflections (160G, 160D, 161G, 161D) of the infrared light source (220, 230, 240) reflected by the two lenses (150G, 150D) of the pair of spectacles (100), and at least one corneal reflection (162D, 162G) of the infrared light source (220, 230, 240) reflected by one of the two eyes of the wearer (1); and
d) deducing, with a processing unit (250):

- said postural characteristic ($\alpha$) depending on the positions of the glass-reflections (160G, 160D, 161G, 161D) in said image (301, 302, 303),
- said behavioural characteristic depending on said postural characteristic ($\alpha$), and
- said geometrico-morphological characteristic (EG, ED, HD, HG) depending on the position of said corneal reflection (162D, 162G) in said image (301, 302, 303).

2. Determining method according to the preceding claim, in which, in step c), in the image (301, 302, 303) acquired in step b), the corneal reflections (162D, 162G) of the infrared light source (220, 230, 240) reflected by both eyes of the wearer (1) are located.

3. Determining method according to one of the preceding claims, in which, in step b), said image (301, 302, 303) is acquired in the domain of the near infrared, with a wavelength comprised between 780 and 1400 nm.

4. Determining method according to one of the preceding claims, in which said postural characteristic comprises a yaw angle ($\alpha$), which separates the sagittal plane (P2) of the head (10) of the wearer (1) and the optical axis (A2) of the infrared image sensor (210).

5. Determining method according to one of the preceding claims, in which said geometrico-morphological characteristic comprises the half pupillary distances (EG, ED) of the wearer (1).

6. Determining method according to one of the preceding claims, in which said geometrico-morphological characteristic comprises the pupillary heights (HG, HD) of the wearer.

7. Determining method according to one of the preceding claims, in which in step b), the infrared image sensor (210) acquires at least two images (301, 302, 303) of the head (10) of the wearer (1), in step c), said reflections (160G, 160D, 161G, 161D) are located in each image (301, 302, 303) acquired in step b), and, in step d), said behavioural characteristic comprises a coefficient quantifying the propensity of the wearer to turn their gaze by moving rather their head or their eyes.

8. Determining method according to one of the preceding claims, in which said geometrico-morphological characteristic comprises the positions of the centres of rotation (OG, OD) of the eyes of the wearer (1).

9. Device (200) for determining a geometrico-morphological characteristic (EG, ED, HD, HG), a postural characteristic ($\alpha$) and a behavioural characteristic of an individual (1), this device comprising:

- at least one infrared light source (220, 230, 240) suitable for illuminating at least part of the head (10) of said individual (1);
- an infrared image sensor (210) suitable for acquiring an image (301, 302, 303) of at least part of the head (10) of said individual (1) illuminated by the infrared light source (220, 230, 240); and
- a processing unit (250) suitable for locating in said image (301, 302, 303) corneal reflections (162D, 162G) of the infrared light source (220, 230, 240) reflected by the pupils of said individual (1) and glass-reflections (160G, 160L, 161G, 161L) of the infrared light source reflected by the two lenses (150D, 150G) of a pair of spectacles (100) worn by said individual (1), and for deducing:
- said postural characteristic ($\alpha$) depending on the positions of the glass-reflections (160G, 160D, 161G, 161D) reflected by the two lenses (150D, 150G),
- said behavioural characteristic depending on said postural characteristic ($\alpha$), and
- the geometrico-morphological characteristic (EG, ED, HD, HG) depending on the position of said corneal reflections (162D, 162G) in said image (301, 302, 303).

10. Determining device according to Claim 9, in which the processing unit is formed by a central processing unit of a computer and in which the infrared light source and the infrared image sensor are separate from the processing unit and are equipped with communicating means suitable for communicating with the central unit of the computer.

11. Determining device (200) according to Claim 9, comprising a supporting structure (201) to which the infrared light source (220, 230, 240), the infrared image sensor (210) and the processing unit (250) are securely fastened.

**12.** Determining device (200) according to the preceding claim, in which the supporting structure (201) comprises a mounting (202) and a slide (205) that is movably mounted on the mounting (202) and that supports the infrared light source (220, 230, 240) and/or the infrared image sensor (210).

**13.** Determining device (200) according to the preceding claim, in which provision is made for means for motorizing the mobility of the slide (205), and in which said processing unit (250) is suitable for automatically controlling said motorizing means to a position such that the face of said individual (1) is visible to the infrared image sensor (210).

**14.** Determining device according to one of Claims 9 to 13, in which provision is made for a single light source mounted movably relative to said image sensor or for at least two light sources that are distant from each other in order to generate two separate reflections in said image.

**15.** Determining device (200) according to one of Claims 9 to 14, in which each infrared light source (220, 230, 240) is composed of at least one light-emitting diode.

**16.** Determining device (200) according to one of Claims 9 to 15, in which the infrared image sensor (210) is also suitable for acquiring images in the visible domain.

**17.** Determining device according to one of Claims 9 to 15, in which provision is made for a visible image sensor that is separate from the infrared image sensor and that is suitable for acquiring an image of the wearer in the visible domain.

**18.** Determining device (200) according to one of Claims 16 and 17, in which provision is made for means for displaying (270) and/or printing (280) the images acquired in the visible domain.

**19.** Determining device (200) according to one of Claims 9 to 18, in which provision is made for means (290) for acquiring at least one dimension of the pair of spectacles (100).

Fig.1

Fig.2

Fig.3

Fig.4

Fig.5

Fig.6

Fig.7

Fig.8

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008009423 A
- WO 2009024681 A
- US 5402199 A
- WO 2008132356 A